(19) Europäisches Patentamt
European Patent Office
Office européen des brevets

(11) EP 4 745 161 A2

(12) EUROPEAN PATENT APPLICATION

(43) Date of publication:
**20.05.2026 Bulletin 2026/21**

(21) Application number: **26158789.3**

(22) Date of filing: **03.04.2018**

(51) International Patent Classification (IPC):
***C07K 16/46*** *(2006.01)*

(52) Cooperative Patent Classification (CPC):
**C07K 16/468; A61K 39/39558; C07K 16/2863; C07K 16/32;** A61K 2039/505; C07K 2317/21; C07K 2317/31; C07K 2317/52

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **31.03.2017 EP 17164382**

(62) Document number(s) of the earlier application(s) in accordance with Art. 76 EPC:
**18717731.6 / 3 600 411**

(71) Applicant: **Merus B.V.**
**3584 CT Utrecht (NL)**

(72) Inventors:
- **THROSBY, Mark**
  **3584 CT Utrecht (NL)**
- **GEUIJEN, Cecilia Anna Wilhelmina**
  **3584 CT Utrecht (NL)**
- **MAUSSANG-DETAILLE, David Andre Baptiste**
  **3584 CT Utrecht (NL)**
- **LOGTENBERG, Ton**
  **3584 CT Utrecht (NL)**

(74) Representative: **V.O.**
**P.O. Box 87930**
**2508 DH Den Haag (NL)**

Remarks:
•The complete document including Reference Table(s) and the Sequence Listing(s) can be downloaded from the EPO website
•This application was filed on 16-02-2026 as a divisional application to the application mentioned under INID code 62.

# (54) ANTIBODIES FOR THE TREATMENT OF ERBB-2/ERBB-3 POSITIVE TUMORS

(57) The invention relates to the field of antibodies. In particular it relates to the field of therapeutic (human) antibodies for the treatment of ErbB-2/ErbB-3 positive tumor. More in particular it relates to treating tumors with a high ErbB-2/ErbB-3 cell-surface receptor ratio. Also encompassed are methods for treating patients not previously treated with an ErbB-2 specific therapy or with an ErbB-3 specific therapy.



Processed by Luminess, 75001 PARIS (FR)

## Description

This application claims priority to EP Application No. 17164382.8, filed March 31, 2017 the contents of which are hereby incorporated by reference.

The invention relates to the field of antibodies. In particular it relates to the field of therapeutic (human) antibodies for the treatment of ErbB-2/ErbB-3 positive tumor. More in particular it relates to treating tumors with a high ErbB-2/ErbB-3 cell-surface receptor ratio. Also encompassed are methods for treating patients not previously treated with an ErbB-2 specific therapy or with a ErbB-3 specific therapy.

The human epidermal growth factor receptor family (HER, also collectively referred to as the ErbB signaling network) is a family of transmembrane receptor tyrosine kinases (RTK). The family includes the epidermal growth factor receptor (EGFR), also known as ErbB-1 (or HERl), and the homologous receptors ErbB-2 (HER2), ErbB-3 (HER3) and ErbB-4 (HER4). The receptors (reviewed in Yarden and Pines 2012) are widely expressed on epithelial cells. Upregulation of HER receptors or their ligands, such as heregulin (HRG) or epidermal growth factor (EGF), is a frequent event in human cancer (Wilson, Fridlyand et al. 2012). Overexpression of ErbB-1 and ErbB-2 in particular occurs in epithelial tumors and is associated with tumor invasion, metastasis, resistance to chemotherapy, and poor prognosis (Zhang, Berezov et al. 2007). In the normal breast, ErbB-3 has been shown to be important in the growth and differentiation of luminal epithelium. For instance, loss/inhibition of ErbB-3 results in selective expansion of the basal over the luminal epithelium (Balko, Miller et al. 2012). Binding of ligand to the extracellular domain of the RTKs induces receptor dimerization, both between the same (homodimerization) and different (heterodimerization) receptor subtypes. Dimerization can activate the intracellular tyrosine kinase domains, which undergo autophosphorylation and, in turn, can activate a number of downstream pro-proliferative signaling pathways, including those mediated by mitogen-activated protein kinases (MAPK) and the prosurvival pathway Akt (reviewed in Yarden and Pines, 2012). No specific endogenous ligand has been identified for ErbB-2, which is therefore assumed to normally signal through heterodimerization (Sergina, Rausch et al. 2007). ErbB-3 can be activated by engagement of its ligands. These ligands include but are not limited to neuregulin (NRG) and heregulin (HRG).

Various modes of activation of signaling of the ErbB receptor family have been identified. Among these are ligand dependent and ligand independent activation of signaling. Over-expressed ErbB-2 is able to generate oncogenic signaling through the ErbB-2:ErbB-3 heterodimer even in the absence of the ErbB-3 ligand (Junttila, Akita et al. 2009). ErbB-2 activity can be inhibited by ErbB-2 specific antibodies. Such ErbB-2 specific antibodies are for instance used in the treatment of ErbB-2 positive (HER2+) tumors. A problem with such treatments is that often tumors escape the ErbB-2 specific treatment and continue to grow even in the presence of the inhibiting antibody. It has been observed that ErbB-2 positive tumors, such as breast, ovarian, cervical and gastric tumors can escape treatment by the selective outgrowth of a subpopulation of tumor cells that exhibit upregulated ErbB-3 expression (Ocana, Vera-Badillo et al. 2013) and/or ErbB-3 ligand expression (Wilson, Fridlyand et al. 2012). Also activating mutations in the ErbB-3 receptor have been identified.

## SUMMARY OF THE INVENTION

In one aspect, a method is provided for the treatment of a subject having an ErbB-2/ErbB-3 positive tumor comprising administering to the subject a bispecific antibody comprising a first antigen-binding site that binds ErbB-2 and a second antigen-binding site that binds ErbB-3, wherein the antibody can reduce a ligand-induced receptor function of ErbB-3 on a ErbB-2 and ErbB-3 positive cell, wherein said ErbB-2/ErbB-3 positive tumor has at least 150,000 ErbB-2 cell-surface receptors per cell, more preferably at least 1,000,000 ErbB-2 cell-surface receptors per cell, and wherein said subject has not previously been treated with a ErbB-2 specific therapy or with a ErbB-3 specific therapy. Preferably, the ErbB-2/ErbB-3 positive tumor has less than 50,000 ErbB-3 cell-surface receptors per cell.

Preferably, the ErbB-2 therapy is an ErbB-2 specific antibody, preferably wherein the ErbB-2 specific antibody is trastuzumab or pertuzumab. Preferably, the ErbB-3 therapy is a ErbB-3 specific antibody, preferably wherein the ErbB-3 specific antibody is MM-121 (seribantumab). Preferably, the method further comprises determining the ErbB-2 cell-surface receptor density for said tumor.

In one aspect a method is provided for the treatment of a subject having an ErbB-2/ErbB-3 positive tumor comprising administering to the subject a bispecific antibody comprising a first antigen-binding site that binds ErbB-2 and a second antigen-binding site that binds ErbB-3, wherein the antibody can reduce a ligand-induced receptor function of ErbB-3 on a ErbB-2 and ErbB-3 positive cell, and wherein said ErbB-2/ErbB-3 positive tumor has a ratio of ErbB-2/ErbB-3 cell-surface receptors per cell of at least 10:1. Preferably, said ErbB-2/ErbB-3 positive tumor has a ratio of ErbB-2/ErbB-3 cell-surface receptors per cell of at least 100:1. Preferably, said ErbB-2/ErbB-3 positive tumor has a ratio of ErbB-2/ErbB-3 cell-surface receptors per cell of at least 3:1. Preferably, the ErbB-2/ErbB-3 positive tumor has at least 150,000 ErbB-2 cell-surface receptors per cell., more preferably at least 1,000,000 ErbB-2 cell-surface receptors per cell.

In one aspect a method is provided for the treatment of a subject having an ErbB-2/ErbB-3 positive tumor comprising administering to the subject a bispecific antibody comprising a first antigen-binding site that binds ErbB-2 and a second antigen-binding site that binds ErbB-3, wherein the antibody can reduce a ligand-induced receptor function of ErbB-3 on a ErbB-2 and ErbB-3 positive cell, and wherein said ErbB-2/ErbB-3 positive tumor has a ratio of Erb-B-1/ErbB-2cell-surface receptors per cell of no more than 6: 10, preferably no more than 4:10, more preferably no more than 2:10. Preferably, the ErbB-2/ErbB-3 positive tumor has at least 150,000 ErbB-2 cell-surface receptors per cell, more preferably at least 1,000,000 ErbB-2 cell-surface receptors per cell. Preferably, the ErbB-2/ErbB-3 positive tumor has no more than 400,000 ErbB-1 cell-surface receptors per cell., more preferably no more than 200,000 ErbB-1 cell-surface receptors per cell. Preferably, the the ErbB-2/ErbB-3 positive tumor has at least 1,000,000 ErbB-2 cell-surface receptors per cell and no more than 200,000 ErbB-1 cell-surface receptors per cell.

In one aspect a method is provided for the treatment of a subject having an ErbB-2/ErbB-3 positive tumor comprising administering to the subject a bispecific antibody comprising a first antigen-binding site that binds ErbB-2 and a second antigen-binding site that binds ErbB-3, wherein the antibody can reduce a ligand-induced receptor function of ErbB-3 on a ErbB-2 and ErbB-3 positive cell, and wherein said ErbB-2/ErbB-3 positive tumor has at least 150,000 ErbB-2 cell-surface receptors per cell, more preferably at least 1,000,000 ErbB-2 cell-surface receptors per cell. Preferably, the ErbB-2/ErbB-3 positive tumor has no more than 400,000 ErbB-1 cell-surface receptors per cell., more preferably no more than 200,000 ErbB-1 cell-surface receptors per cell. Preferably, the the ErbB-2/ErbB-3 positive tumor has at least 1,000,000 ErbB-2 cell-surface receptors per cell and no more than 200,000 ErbB-1 cell-surface receptors per cell. Preferably, the the ErbB-2/ErbB-3 positive tumor has at least 1,000,000 ErbB-2 cell-surface receptors per cell and no more than 400,000 ErbB-1 cell-surface receptors per cell.

Preferably in the methods disclosed herein, the ErbB-2/ErbB-3 positive tumor has less than 50,000 ErbB-3 cell-surface receptors per cell.

Preferably in the methods disclosed herein, the cells of said tumor have a heregulin expression level that is greater than the heregulin expression level of MCF7 cells.

As is clear to a skilled person the bispecific antibodies disclosed herein are also for the use in the preparation of a medicament and for the use in therapy, as disclosed herein. In particular, the bispecific antibodies are for use in the treatment of an ErbB-2/ErbB-3 positive tumor, wherein said ErbB-2/ErbB-3 positive tumor has at least 150,000 ErbB-2 cell-surface receptors per cell, more preferably at least 1,000,000 ErbB-2 cell-surface receptors per cell, and wherein said treatment is for a subject that has not previously been treated with a ErbB-2 specific therapy or with a ErbB-3 specific therapy. Furthermore, the bispecific antibodies are for use in the treatment of an ErbB-2/ErbB-3 positive tumor, wherein said ErbB-2/ErbB-3 positive tumor has a ratio of ErbB-2/ErbB-3 cell-surface receptors per cell of at least 10:1. Furthermore, the bispecific antibodies are for use in the treatment of an ErbB-2/ErbB-3 positive tumor, wherein said ErbB-2/ErbB-3 positive tumor has a ratio of ErbB-1/ErbB-2 cell-surface receptors per cell of no more than 6: 10, preferably no more than 4:10, more preferably no more than 2:10.

Preferably in the methods disclosed herein, said first antigen-binding site binds domain I of ErbB-2 and said second antigen-binding site binds domain III of ErbB-3, preferably wherein the affinity of the first antigen-binding site for ErbB-2 is lower than the affinity of the second antigen-binding site for ErbB-3. Preferably wherein said antibody comprises

i) at least the CDR1, CDR2 and CDR3 sequences of an ErbB-2 specific heavy chain variable region selected from the group consisting of MF2926, MF2930, MF1849; MF2973, MF3004, MF3958, MF2971, MF3025, MF2916, MF3991, MF3031, MF2889, MF2913, MF1847, MF3001, MF3003 and MF1898 or wherein said antibody comprises CDR sequences that differ in at most 3 amino acids, preferably in at most 2 amino acids, preferably in at most 1 amino acid from the CDR1, CDR2 and CDR3 sequences of MF2926, MF2930, MF1849; MF2973, MF3004, MF3958, MF2971, MF3025, MF2916, MF3991, MF3031, MF2889, MF2913, MF1847, MF3001, MF3003 or MF1898; and/or

ii) at least the CDR1, CDR2 and CDR3 sequences of an ErbB-3 specific heavy chain variable region selected from the group consisting of MF3178; MF3176; MF3163; MF3099; MF3307; MF6055; MF6056; MF6057; MF6058; MF6059; MF6060; MF6061; MF6062; MF6063; MF6064; MF 6065; MF6066; MF6067; MF6068; MF6069; MF6070; MF6071; MF6072; MF6073 and MF6074, or wherein said antibody comprises CDR sequences that differ in at most 3 amino acids, preferably in at most 2 amino acids, preferably in at most 1 amino acid from the CDR1, CDR2 and CDR3 sequences of MF3178; MF3176; MF3163; MF3099; MF3307; MF6055; MF6056; MF6057; MF6058; MF6059; MF6060; MF6061; MF6062; MF6063; MF6064; MF 6065; MF6066; MF6067; MF6068; MF6069; MF6070; MF6071; MF6072; MF6073 or MF6074. Preferably, the antibody comprises

i) an ErbB-2 specific heavy chain variable region sequence selected from the group consisting of the heavy chain variable region sequences of MF2926, MF2930, MF1849; MF2973, MF3004, MF3958, MF2971, MF3025, MF2916, MF3991, MF3031, MF2889, MF2913, MF1847, MF3001, MF3003 and MF1898, or wherein said antibody comprises a heavy chain variable region sequence that differs in at most 15 amino acids from the heavy chain variable region sequences of MF2926, MF2930, MF1849; MF2973, MF3004, MF3958, MF2971, MF3025, MF2916, MF3991, MF3031, MF2889, MF2913, MF1847, MF3001, MF3003 or MF1898; and/or

ii) an ErbB-3 specific heavy chain variable region sequence selected from the group consisting of the heavy chain variable region sequences of MF3178; MF3176; MF3163; MF3099; MF3307; MF6055; MF6056; MF6057; MF6058; MF6059; MF6060; MF6061; MF6062; MF6063; MF6064; MF 6065; MF6066; MF6067; MF6068; MF6069; MF6070; MF6071; MF6072; MF6073 and MF6074, or wherein said antibody comprises a heavy chain variable region sequence that differs in at most 15 amino acids from the heavy chain variable region sequences of MF3178; MF3176; MF3163; MF3099; MF3307; MF6055; MF6056; MF6057; MF6058; MF6059; MF6060; MF6061; MF6062; MF6063; MF6064; MF 6065; MF6066; MF6067; MF6068; MF6069; MF6070; MF6071; MF6072; MF6073 or MF6074. Preferably, the antibody comprises at least the CDR1, CDR2 and CDR3 sequences of the ErbB-2 specific heavy chain variable region MF3958 and the antibody comprises at least the CDR1, CDR2 and CDR3 sequences of the ErbB-3 specific heavy chain variable region MF3178. Preferably, the bispecific antibody comprises the "heavy chain for erbB-2 binding" as depicted in the Sequence listing part 1D and the "heavy chain for erbB-3 binding" as depicted in the Sequence listing part 1D.

Preferably, the first antigen binding site and said second antigen binding site comprise a light chain variable region comprising the IgVKI-39 gene segment, most preferably the rearranged germline human kappa light chain IgVKI-39*01/IGJKI*01 or IgVκ1-39*01/IGJκ5*01. Preferably, the light chain variable region comprises a CDR1 having the sequence (RASQSISSYLN), a CDR2 having the sequence (AASSLQS), and a CDR3 having the sequence (QQSYSTPPT).

DETAILED DESCRIPTION OF THE INVENTION

The invention provides methods of treating a subject having an ErbB-2/ErbB-3 positive tumor with a bispecific antibody disclosed herein, wherein said ErbB-2/ErbB-3 positive tumor has at least 150,000 ErbB-2 cell-surface receptors per cell, preferably at least 1,000,000 ErbB-2 cell-surface receptors per cell, and wherein said subject has not previously been treated with a ErbB-2 specific therapy or with a ErbB-3 specific therapy.

The invention also provides methods of treating a subject having an ErbB-2/ErbB-3 positive tumor with a bispecific antibody disclosed herein, wherein said ErbB-2/ErbB-3 positive tumor has a ratio of ErbB-2/ErbB-3 cell-surface receptors per cell of at least 10:1. Preferably, said ErbB-2/ErbB-3 positive tumor has a ratio of ErbB-2/ErbB-3 cell-surface receptors per cell of at least 100:1 or at least 1,000:1.

As exemplified in Figure 4, the bispecific antibodies disclosed herein are more effective at inhibiting HRG - HER3 binding than monospecific HER3 antibodies under high heregulin stress conditions and/or when HER2 levels are greater than HER3 levels. Without wishing to be bound by theory, this effect may be due to the enhanced ability of the bispecific antibodies to target ErbB-2/ErbB-3 tumors over monospecific HER3 antibodies *in vivo.* This "ErbB-2 guided targeting" is also demonstrated in Figure 2, which demonstrates that the ErbB-2 specific arm of the disclosed bispecific antibodies is responsible for the enhanced binding on tumor cells. While not wishing to be bound by theory, these results support the treatment of specific patient populations with the bispecific antibodies. Preferred patient populations have high ErbB-2 levels in comparison to ErbB-3 levels. It is known that one of the mechanisms of escape for tumors in response to treatment with an ErbB-2 specific therapy is to upregulate ErbB-3 levels. Thus, preferred patient populations treated with the disclosed bispecifics have not been previously treated with an ErbB-2 specific therapy or with a ErbB-3 specific therapy. Often such tumors do not have upregulated ErbB-3 levels. An antibody of the invention is particularly suited to target such tumors and thereby reduce the escape potential of such tumors.

The invention also provides methods of treating a subject having an ErbB-2/ErbB-3 positive tumor with a bispecific antibody disclosed herein, wherein the tumor has a ratio of ErbB-1/ErbB-2 cell-surface receptors per cell of no more than 6: 10, preferably no more than 4:10, more preferably no more than 2:10.

To establish whether a tumor is positive for ErbB-2 and ErbB-3 the skilled person can for instance determine the ErbB-2 and ErbB-3 amplification and/or staining in immunohistochemistry. At least 10% tumor cells in a biopsy should be positive for both ErbB-2 and for ErbB-3. The biopsy can also contain 20%, 30% 40% 50% 60% 70% or more positive cells. ErbB-1 positive tumors can be similarly identified.

Preferably said positive cancer is a breast cancer, such as early-stage breast cancer. However, the invention can be applied to a wide range of ErbB-2, ErbB-3 or ErbB-2/ErbB-3 positive cancers, like gastric cancer, colorectal cancer, colon cancer, gastro-esophageal cancer, esophageal cancer, endometrial cancer, ovarian cancer, liver cancer, lung cancer including non-small cell lung cancer, clear cell sarcoma, salivary gland cancer, head and neck cancer, brain cancer, bladder cancer, pancreatic cancer, prostate cancer, kidney cancer, skin cancer, melanoma, and the like.

Patients with ErbB 2 positive tumor cells can be classified based on the number of ErbB-2 receptors on the tumor cell surface. Tumors with more than 1,000,000 ErbB-2 receptors on their cell surface are typically classified as ErbB-2 [++ +], those with between 150.000 to 1,000,000 are classified as ErbB-2 [++], and those with less than 150,000 are classified as ErbB-2[+]. Preferably, the patient is classified as ErbB-2[++] or ErbB-2 [+++]. Preferably, the ErbB-2/ErbB-3 positive tumor has at least 1,000,000 ErbB-2 cell-surface receptors per cell.

Preferably, methods are provided in which the ErbB-2/ErbB-3 positive tumor has a ratio of ErbB-2/ErbB-3 cell-surface receptors per cell of at least 10:1 and the ErbB-2/ErbB-3 positive tumor has at least 150,000 ErbB-2 cell-surface receptors per cell;

the ErbB-2/ErbB-3 positive tumor has a ratio of ErbB-2/ErbB-3 cell-surface receptors per cell of at least 10:1 and the ErbB-2/ErbB-3 positive tumor has at least 1,000,000 ErbB-2 cell-surface receptors per cell;
the ErbB-2/ErbB-3 positive tumor has a ratio of ErbB-2/ErbB-3 cell-surface receptors per cell of at least 100:1 and the ErbB-2/ErbB-3 positive tumor has at least 150,000 ErbB-2 cell-surface receptors per cell;
the ErbB-2/ErbB-3 positive tumor has a ratio of ErbB-2/ErbB-3 cell-surface receptors per cell of at least 100:1 and the ErbB-2/ErbB-3 positive tumor has at least 1,000,000 ErbB-2 cell-surface receptors per cell;
the ErbB-2/ErbB-3 positive tumor has a ratio of ErbB-2/ErbB-3 cell-surface receptors per cell of at least 1000:1 and the ErbB-2/ErbB-3 positive tumor has at least 150,000 ErbB-2 cell-surface receptors per cell; or
the ErbB-2/ErbB-3 positive tumor has a ratio of ErbB-2/ErbB-3 cell-surface receptors per cell of at least 1000:1 and the ErbB-2/ErbB-3 positive tumor has at least 1,000,000 ErbB-2 cell-surface receptors per cell.

Preferably, the subject has not previously been treated with a ErbB-2 specific therapy or with a ErbB-3 specific therapy.
Preferably, methods are provided in which the ErbB-2/ErbB-3 positive tumor has at least 150,000 ErbB-2 cell-surface receptors per cell and less than 50,000 ErbB-3 cell-surface receptors per cell. Preferably, methods are provided in which the ErbB-2/ErbB-3 positive tumor has at least 1,00,000 ErbB-2 cell-surface receptors per cell and less than 50,000 ErbB-3 cell-surface receptors per cell.
Preferably, methods are provided in which the ErbB-2/ErbB-3 positive tumor has a ratio of ErbB-1/ErbB-2 cell-surface receptors per cell of no more than 6:10 and the ErbB-2/ErbB-3 positive tumor has at least 150,000 ErbB-2 cell-surface receptors per cell;

the ErbB-2/ErbB-3 positive tumor has a ratio of ErbB-1/ErbB-2 cell-surface receptors per cell of no more than 6:10 and the ErbB-2/ErbB-3 positive tumor has at least 1,000,000 ErbB-2 cell-surface receptors per cell;
the ErbB-2/ErbB-3 positive tumor has a ratio of ErbB-1/ErbB-2 cell-surface receptors per cell of no more than 4:10 and the ErbB-2/ErbB-3 positive tumor has at least 150,000 ErbB-2 cell-surface receptors per cell;
the ErbB-2/ErbB-3 positive tumor has a ratio of ErbB-1/ErbB-2 cell-surface receptors per cell of no more than 4:10 and the ErbB-2/ErbB-3 positive tumor has at least 1,000,000 ErbB-2 cell-surface receptors per cell;
the ErbB-2/ErbB-3 positive tumor has a ratio of ErbB-1/ErbB-2 cell-surface receptors per cell of no more than 2:10 and the ErbB-2/ErbB-3 positive tumor has at least 150,000 ErbB-2 cell-surface receptors per cell; or
the ErbB-2/ErbB-3 positive tumor has a ratio of ErbB-1/ErbB-2 cell-surface receptors per cell of no more than 2:10 and the ErbB-2/ErbB-3 positive tumor has at least 1,000,000 ErbB-2 cell-surface receptors per cell.
Preferably, the subject has not previously been treated with a ErbB-2 specific therapy or with a ErbB-3 specific therapy.

In some embodiments, the methods disclosed herein are advantageous in that specific patient populations are first determined based on, e.g., the ErbB-1, ErbB-2, and/or ErbB-3 cell-surface receptor density. While not wishing to be bound by theory, such patient stratification is expected to identify patients with the greatest likelihood of responding to the bispecific antibodies. As is well-known to a skilled person, cancer therapeutics can have significant side-effects. One object of the invention is to avoid the treatment of patients that are likely not to benefit from the bispecific antibodies. Accordingly, the methods disclosed herein preferably comprise determining the ErbB-1 cell-surface receptor density, ErbB-2 cell-surface receptor density, and/or ErbB-3 cell-surface receptor density for said tumor. Preferably, the ErbB-1 cell-surface receptor density and ErbB-2 cell-surface receptor density are determined. As used herein, the term cell-surface receptors density refers to the number of receptors present at the cell-surface per cell.
Preferably, the methods disclosed herin further comprise determining the ErbB-2 or ErbB-3 cell-surface receptor density for said tumor. Patients may be classified using immunocytochemistry or fluorescence *in situ* hybridization. The HercepTest™ and/or HER2 FISH (pharm Dx™), marketed both by Dako Denmark A/S, and/or using a HERmark® assay, marketed by Monogram Biosciences are examples of suitable assays for determining ErbB-2 or ErbB-3 cell surface receptor density. Other methods for determining the ErbB-2 receptor cell density are well-known to a skilled person. In vivo methods for determining ErbB-2 are also known, see, e.g., Chernomoridik et al. Mol Imaging. 2010 Aug; 9(4): 192-200 and Ardeshirpour et al. Technol Cancer Res Treat. 2014 Oct; 13(5): 427-434.
Preferably, the methods disclosed herin further comprise determining the ErbB-2 cell-surface receptor density for said tumor. Such methods are known to a skilled person (see, e.g., van der Woning and van Zoelen Biochem Biophys Res Commun. 2009 Jan 9;378(2):285-9).
Preferably, the methods disclosed herin further comprise determining the ErbB-1 cell-surface receptor density for said tumor. Such methods are known to a skilled person (see, e.g., EGFR pharmDx™Kit (Dako)) amd McDonagh et al. Mol Cancer Ther 2012; 11:582).
In some embodiments, the ErbB-1, ErbB-2, and ErbB-3 cell-surface receptor densities are determined by FACS analysis on biopsied tumor cells.

It is clear to a skilled person that the term "treated with a ErbB-2 specific therapy" refers to a treatment of the patient's tumor. ErbB-2 specific therapies are well-known to a skilled person. As used herein, an ErbB-2 specific therapy refers to a treatment that specifically reduces the expression and/or activity of ErbB-2 in a tumor. Such ErbB-2 inhibitors include, e.g., nucleic acid molecules (e.g., RNAi, antisense oligonucleotides, siRNA) and small molecule inhibitors (e.g., lapatinib, afatinib, neratinib, canertinib, irbinitinib, CP-724714, mubritinib, and afatinibi). Preferably the ErbB-2 specific therapy is an ErbB-2 specific antibody such as trastuzumab or pertuzumab. Currently used therapies such as trastuzumab (Herceptin) and pertuzumab are only prescribed for patients with malignant ErbB 2 positive cells that have more than 1.000.000 ErbB-2 receptors on their cell surface, in order to obtain a clinical response. Trastuzumab and pertuzumab are only prescribed to ErbB-2 [+++] patients because patients with lower ErbB-2 concentrations typically do not exhibit a sufficient clinical response when treated with trastuzumab and pertuzumab. While not wishing to be bound by theory, previous treatment with an ErbB-2 specific therapy is believed to often result in the upregulation of ErbB-3.

It is clear to a skilled person that the term "treated with a ErbB-3 specific therapy" refers to a treatment of the patient's tumor. As used herein, an ErbB-3 specific therapy refers to a treatment that specifically reduces the expression and/or activity of ErbB-3 in a tumor. Such ErbB-3 inhibitors include an ErbB-3 specific antibody such as MM-121 (seribantumab).

Preferably, the cells of the ErbB-2/ErbB-3 positive tumor have relatively high levels of heregulin expression. Heregulin is a growth factor that is involved in growth of ErbB 3 positive tumor cells. Typically, when the tumor cells express high levels of heregulin (referred to as heregulin stress), currently known therapies like trastuzumab, pertuzumab and lapatinib are no longer capable of inhibiting tumor growth. This phenomenon is called heregulin resistance. In particular, the heregulin expression level that is greater than the heregulin expression level of MCF7 cells. Heregulin expression levels are for instance measured using qPCR with tumor RNA (such as for instance described in Shames et al. PLOS ONE, February 2013, Vol.8, Issue 2, pp 1-10 and in Yonesaka et al., Sci.transl.Med., Vol.3, Issue 99 (2011); pp1-11), or using protein detection methods, like for instance ELISA, preferably using blood, plasma or serum samples (such as for instance described in Yonesaka et al., Sci.transl.Med., Vol.3, Issue 99 (2011); pp1-11).

High heregulin levels are typically present during the formation of metastases (i.e. the migration, invasion, growth and/or differentiation of tumor cells or tumor initiating cells). Typically, tumor initiating cells are identified based on stem cell markers such as for instance CD44, CD24, CD133 and/or ALDH1. These processes can therefore barely be counteracted with currently known therapies like trastuzumab and pertuzumab. The bispecific antibodies disclosed herein are capable of counteracting the formation of metastases in subjects that have not previously been treated with a ErbB-2 specific therapy or with a ErbB-3 specific therapy and have an ErbB-2 cell-receptor density as described herein. The bispecific antibodies disclosed herein are are also capable of counteracting the formation of metastases in subjects having a ratio of ErbB-2/ErbB-3 cell-surface receptors per cell and/or a ratio of ErbB-1/ErbB-2 cell-surface receptors per cell as disclosed herein.

The subject is preferably a human subject. The subject is preferably a subject eligible for monoclonal antibody therapy using an ErbB-2 specific antibody such as trastuzumab.

The amount of bispecific to be administered to a patient is typically in the therapeutic window, meaning that a sufficient quantity is used for obtaining a therapeutic effect, while the amount does not exceed a threshold value leading to an unacceptable extent of side-effects. The lower the amount of antibody needed for obtaining a desired therapeutic effect, the larger the therapeutic window will typically be. The selected dosage level will depend upon a variety of factors including the route of administration, the time of administration, the rate of excretion of the particular compound being employed, the duration of the treatment, other drugs, compounds and/or materials used in combination, the age, sex, weight, condition, general health and prior medical history of the patient being treated, and like factors well known in the medical arts. The dosage can be in the range of the dosing regime for trastuzumab or lower.

The bispecific antibodies can be formulated as a pharmaceutical composition comprising pharmaceutically acceptable carrier, diluent, or excipient. and additional, optional, active agents. The antibodies and compositions comprising the antibodies can be administered by any route including parenteral, enteral, and topical administration. Parenteral administration is usually by injection, and includes, e.g., intravenous, intramuscular, intraarterial, intrathecal, intraventricular, intracapsular, intraorbital, intracardiac, intradermal, intraperitoneal, transtracheal, subcutaneous, subcuticular, intraarticular, sub capsular, subarachnoid, intraspinal, intracerebro spinal, intratumoral, and intrasternal injection and infusion.

**In** preferred embodiments, an ErbB-1 inhibitor can be combined with treatment with the bispecific antibodies disclsosed herein. The ErbB-1 inhibitor can be administered simulateously or sequentially with the bispecific antibody. Treatment with the ErbB-1 inhibitor can be separated by several minutes, hours, or days from the treatment with the bispecific antibody. Preferably, the ErbB-2/ErbB3 tumor is also positive for ErbB1. Preferably, the combination treatment is suitable for ErbB-2/ErbB3 tumors having more than 5,000 surface receptors per cell, preferably at least 20,000 surface receptors per cell, more preferably more than 50,000 surface receptors per cell.

Suitable ErbB-1 inhibitors are known in the art and refer to compounds that inhibit at least one biological activity of ErbB-1 (EGFR), in particular a compound that decreases the expression or signaling activity of ErbB-1. Preferred ErbB-1 inhibitors bind to the extracellular binding site of the tyrosine kinase receptor molecule and block binding of the natural ligands, such as EGF. Such inhibitors include antibodies, antibody portions, and peptides comprising epitopes that target this extracellular EGF receptor binding domain. Preferably, the ErbB-1 inhibitor is an anti-ErbB-1 antibody, preferably selected from cetuximab, matuzumab, necitumumab, nimotuzumab, panitumumab, or zalutumumab. The invention is further related to ErbB-1 inhibitors which can bind or interact with the intracellular phosphorylation site or domain of the tyrosine kinase receptor molecule, such preventing or decreasing phosphorylation by tyrosine kinase. This can be achieved by small (chemical) molecule drugs. Preferred inhibitors include afatinib, erlotinib, gefitinib, lapatinib, osimertinib, and neratinib.

The disclosure provides bispecific antibodies for use in the methods and treatments described herein. Suitable bispecific antibodies comprise a first antigen-binding site that binds ErbB-2 and a second antigen-binding site that binds ErbB-3, wherein the bispecific antibody reduces or can reduce a ligand-induced receptor function of ErbB-3 on a ErbB-2 and ErbB-3 positive cell. Preferred antibodies and their preparation are disclosed in WO 2015/130173, which is hereby incorporated by reference. The examples in WO 2015/130173 further describe a number of properties of the antibodes, such as ligand binding and epitope mapping.

As used herein, the term "antigen-binding site" refers to a site derived from and preferably as present on a bispecific antibody which is capable of binding to antigen. An unmodified antigen-binding site is typically formed by and present in the variable domain of the antibody. The variable domain contains said antigen-binding site. A variable domain that binds an antigen is a variable domain comprising an antigen-binding site that binds the antigen.

In one embodiment an antibody variable domain comprises a heavy chain variable region (VH) and a light chain variable region (VL). The antigen-binding site can be present in the combined VH/VL variable domain, or in only the VH region or only the VL region. When the antigen-binding site is present in only one of the two regions of the variable domain, the counterpart variable region can contribute to the folding and/or stability of the binding variable region, but does not significantly contribute to the binding of the antigen itself.

As used herein, antigen-binding refers to the typical binding capacity of an antibody to its antigen. An antibody comprising an antigen-binding site that binds to ErbB-2, binds to ErbB-2 and, under otherwise identical conditions, at least 100-fold lower to the homologous receptors ErbB-1 and ErbB-4 of the same species. An antibody comprising an antigen-binding site that binds to ErbB-3, binds to ErbB-3 and, under otherwise identical conditions, not to the homologous receptors ErbB-1 and ErbB-4 of the same species. Considering that the ErbB-family is a family of cell surface receptors, the binding is typically assessed on cells that express the receptor(s). Binding of an antibody to an antigen can be assessed in various ways. One way is to incubate the antibody with the antigen (preferably cells expressing the antigen), removing unbound antibody (preferably by a wash step) and detecting bound antibody by means of a labeled antibody that binds to the bound antibody.

Antigen binding by an antibody is typically mediated through the complementarity regions of the antibody and the specific three-dimensional structure of both the antigen and the variable domain allowing these two structures to bind together with precision (an interaction similar to a lock and key), as opposed to random, non-specific sticking of antibodies. As an antibody typically recognizes an epitope of an antigen, and as such epitope may be present in other compounds as well, antibodies according to the present invention that bind ErbB-2 and/or ErbB-3 may recognize other proteins as well, if such other compounds contain the same epitope. Hence, the term "binding" does not exclude binding of the antibodies to another protein or protein(s) that contain the same epitope. Such other protein(s) is preferably not a human protein. An ErbB-2 antigen-binding site and an ErbB-3 antigen-binding site as defined herein typically do not bind to other proteins on the membrane of cells in a post-natal, preferably adult human. A bispecific antibody as disclosed herein is typically capable of binding ErbB-2 and ErbB-3 with a binding affinity of at least 1x10e-6 M, as outlined in more detail below.

The term "interferes with binding" as used herein means that the antibody is directed to an epitope on ErbB-3 and the antibody competes with ligand for binding to ErbB-3. The antibody may diminish ligand binding, displace ligand when this is already bound to ErbB-3 or it may, for instance through steric hindrance, at least partially prevent that ligand can bind to ErbB-3.

The term "antibody" as used herein means a proteinaceous molecule, preferably belonging to the immunoglobulin class of proteins, containing one or more variable domains that bind an epitope on an antigen, where such domains are derived from or share sequence homology with the variable domain of an antibody. Antibodies for therapeutic use are preferably as close to natural antibodies of the subject to be treated as possible (for instance human antibodies for human subjects). Antibody binding can be expressed in terms of specificity and affinity. The specificity determines which antigen or epitope thereof is specifically bound by the binding domain. The affinity is a measure for the strength of binding to a particular antigen or epitope. Specific binding, is defined as binding with affinities (KD) of at least 1x10e-6 M, more preferably 1x10e-7 M, more preferably higher than 1x10e-9 M. Typically, antibodies for therapeutic applications have affinities of up to 1x10e-10 M or higher. Antibodies such the bispecific antibodies of the present invention comprise the constant domains (Fc part) of a natural antibody. An antibody of the invention is typically a bispecific full length antibody, preferably of the human IgG subclass. Preferably, an antibody as disclosed herein is of the human IgG1 subclass. Such antibodies have good ADCC properties, have favorable half life upon in vivo administration to humans and CH3 engineering technology exists that can provide for modified heavy chains that preferentially form heterodimers over homodimers upon co-expression in clonal cells.

An antibody as disclosed herein is preferably a "full length" antibody. The term 'full length' is defined as comprising an essentially complete antibody, which however does not necessarily have all functions of an intact antibody. For the avoidance of doubt, a full length antibody contains two heavy and two light chains. Each chain contains constant (C) and variable (V) regions, which can be broken down into domains designated CH1, CH2, CH3, VH, and CL, VL. An antibody binds to antigen via the variable domains contained in the Fab portion, and after binding can interact with molecules and cells of the immune system through the constant domains, mostly through the Fc portion. The terms 'variable domain', 'VH/VL pair', 'VH/VL' are used herein interchangeably. Full length antibodies according to the invention encompass antibodies wherein mutations may be present that provide desired characteristics. Such mutations should not be deletions of substantial portions of any of the regions. However, antibodies wherein one or several amino acid residues are deleted, without essentially altering the binding characteristics of the resulting antibody are embraced within the term "full length antibody". For instance, an IgG antibody can have 1-20 amino acid residue insertions, deletions or a combination thereof in the constant region. For instance, ADCC activity of an antibody can be improved when the antibody itself has a low ADCC activity, by slightly modifying the constant region of the antibody (Junttila, T. T., K. Parsons, et al. (2010). "Superior In vivo Efficacy of Afucosylated Trastuzumab in the Treatment of HER2-Amplified Breast Cancer." Cancer Research 70(11): 4481-4489)

Full length IgG antibodies are preferred because of their favourable half life and the need to stay as close to fully autologous (human) molecules for reasons of immunogenicity. An antibody as disclosed herein is preferably a bispecific IgG antibody, preferably a bispecific full length IgG1 antibody. IgG1 is favoured based on its long circulatory half life in man. In order to prevent any immunogenicity in humans it is preferred that the bispecific IgG antibody is a human IgG1.

The term 'bispecific' (bs) means that one part of the antibody (as defined above) binds to one epitope on an antigen whereas a second part binds to a different epitope. The different epitope is typically present on a different antigen. The first and second antigens are in fact two different proteins. A preferred bispecific antibody is an antibody that comprises parts of two different monoclonal antibodies and consequently binds to two different types of antigen. One arm of the bispecific antibody typically contains the variable domain of one antibody and the other arm contains the variable domain of another antibody. The heavy chain variable regions of the bispecific antibody are typically different from each other, whereas the light chain variable regions are preferably the same. A bispecific antibody wherein the different heavy chain variable regions are associated with the same, or a common, light chain is also referred to as a bispecific antibody with a common light chain.

Preferred bispecific antibodies can be obtained by co-expression of two different heavy chains and a common light chain in a single cell. When wildtype CH3 domains are used, co-expression of two different heavy chains and a common light chain will result in three different species, AA, AB and BB. To increase the percentage of the desired bispecific product (AB) CH3 engineering can be employed, or in other words, one can use heavy chains with compatible heterodimerization domains, as defined hereunder.

The term 'compatible heterodimerization domains' as used herein refers to protein domains that are engineered such that engineered domain A' will preferentially form heterodimers with engineered domain B' and vice versa, whereas homodimerization between A'-A' and B'-B' is diminished.

The term 'common light chain' refers to light chains which may be identical or have some amino acid sequence differences while the binding specificity of the full length antibody is not affected . It is for instance possible, to prepare or find light chains that are not identical but still functionally equivalent, e.g., by introducing and testing conservative amino acid changes, changes of amino acids in regions that do not or only partly contribute to binding specificity when paired with the heavy chain, and the like. The terms 'common light chain', 'common VL', 'single light chain', 'single VL', with or without the addition of the term 'rearranged' are all used herein interchangeably.

A common light chain (variable region) preferably has a germline sequence. A preferred germline sequence is a light chain variable region that is frequently used in the human repertoire and has good thermodynamic stability, yield and solubility. In a preferred embodiment the light chain comprises a light chain region comprising the amino acid sequence of an 012 / IgVκ1-39*01 gene segment as depicted in the Sequences 1C "Common light chain IGKV1-39/jk1" with 0-10, preferably 0-5 amino acid insertions, deletions, substitutions, additions or a combination thereof. IgVκ1-39 is short for Immunoglobulin Variable Kappa 1-39 Gene. The gene is also known as Immunoglobulin Kappa Variable 1-39; IGKV139; IGKV1-39; O12a or 012. External Ids for the gene are HGNC: 5740; Entrez Gene: 28930; Ensembl: ENSG00000242371. The variable region of IGKV1-39 is listed in the Sequences 1C. The V-region can be combined with one of five J-regions. Sequences 1C describe two preferred sequences for IgVκ1-39 in combination with a J-region. The joined sequences are indicated as IGKV1-39/jk1 and IGKV1-39/jk5; alternative names are IgVκ1-39*01/IGJκ1*01 or 1gVκ1-39*01/IGJκ5*01 (nomenclature according to the IMGT database worldwide web at imgt.org).

It is preferred that the O12 / IgVκ1-39*01 comprising light chain variable region is a germline sequence. It is further preferred that the IGJκ1*01 or /IGJκ5*01 comprising light chain variable region is a germline sequence. In a preferred embodiment, the IGKV1-39/jk1 or IGKV1-39/jk5 light chain variable regions are germline sequences.

In a preferred embodiment the light chain variable region comprises a germline 012 / IgVκ1-39*01. In a preferred embodiment the light chain variable region comprises the kappa light chain IgVκ1-39*01/I1GJκ1*01 or IgVκ1-39*01/IGJκ5*01. In a preferred embodiment a IgVκ1-39*01/IGJκ1*01. The light chain variable region preferably comprises a germline kappa light chain IgVκ1-39*01/IGJκ1*01 or germline kappa light chain IgVκ1-39*01/1GJκ5*01, preferably a germline IgVκ1-39*01/IGJκ1*01.

Obviously, those of skill in the art will recognize that "common" also refers to functional equivalents of the light chain of which the amino acid sequence is not identical. Many variants of said light chain exist wherein mutations (deletions, substitutions, additions) are present that do not materially influence the formation of functional binding regions. The light chain can also be a light chain as specified herein above, having 1-5 amino acid insertions, deletions, substitutions or a combination thereof.

Preferably, both the first antigen binding site and said second antigen binding site comprise a light chain variable region comprising a CDR1 having the sequence (RASQSISSYLN), a CDR2 having the sequence (AASSLQS), and a CDR3 having the sequence (QQSYSTPPT).

The term 'ErbB-1' as used herein refers to the protein that in humans is encoded by the ERBB-1 gene. Alternative names for the gene or protein include EGFR, ERBB, HER1, Erb-B2 receptor tyrosine kinase 1. Where reference is made herein to ErbB-1, the reference refers to human ErbB-1.

The term 'ErbB-2' as used herein refers to the protein that in humans is encoded by the ERBB-2 gene. Alternative names for the gene or protein include CD340; HER-2; HER-2/neu; MLN 19; NEU; NGL; TKR1. The ERBB-2 gene is frequently called HER2 (from human epidermal growth factor receptor 2). Where reference is made herein to ErbB-2, the reference refers to human ErbB-2. An antibody comprising an antigen-binding site that binds ErbB-2, binds human ErbB-2. The ErbB-2 antigen-binding site may, due to sequence and tertiary structure similarity between human and other mammalian orthologs, also bind such an ortholog but not necessarily so. Database accession numbers for the human ErbB-2 protein and the gene encoding it are (NP_001005862.1, NP_004439.2 NC_000017.10 NT_010783.15 NC_018928.2). The accession numbers are primarily given to provide a further method of identification of ErbB-2 as a target, the actual sequence of the ErbB-2 protein bound the antibody may vary, for instance because of a mutation in the encoding gene such as those occurring in some cancers or the like. The ErbB-2 antigen binding site binds ErbB-2 and a variety of variants thereof, such as those expressed by some ErbB-2 positive tumor cells.

The term 'ErbB-3' as used herein refers to the protein that in humans is encoded by the ERBB-3 gene. Alternative names for the gene or protein are HER3; LCCS2; MDA-BF-1; c-ErbB-3; c-erbb-3; erbb-3-S; p180-Erbb-3; p45-sErbb-3; and p85-sErbb-3. Where reference is made herein to ErbB-3, the reference refers to human ErbB-3. An antibody comprising an antigen-binding site that binds ErbB-3, binds human ErbB-3. The ErbB-3 antigen-binding site, may, due to sequence and tertiary structure similarity between human and other mammalian orthologs, also bind such an ortholog but not necessarily so. Database accession numbers for the human ErbB-3 protein and the gene encoding it are (NP_001005915.1 NP_001973.2, NC_000012.11 NC_018923.2 NT_029419.12 ). The accession numbers are primarily given to provide a further method of identification of ErbB-3 as a target, the actual sequence of the ErbB-3 protein bound by an antibody may vary, for instance because of a mutation in the encoding gene such as those occurring in some cancers or the like. The ErbB-3 antigen binding site binds ErbB-3 and a variety of variants thereof, such as those expressed by some ErbB-2 positive tumor cells.

The antibodies disclosed herein can reduce a ligand-induced receptor function of ErbB-3 on an ErbB-2 and ErbB-3 positive cell. In the presence of excess ErbB-2, ErbB-2/ErbB-3 heterodimers may provide a growth signal to the expressing cell in the absence of detectable ligand for the ErbB-3 chain in the heterodimer. This ErbB-3 receptor function is herein referred as a ligand-independent receptor function of ErbB-3. The ErbB-2/ErbB-3 heterodimer also provide a growth signal to the expressing cell in the presence of an ErbB-3 ligand. This ErbB-3 receptor function is herein referred to as a ligand-induced receptor function of ErbB-3.

The term "ErbB-3 ligand" as used herein refers to polypeptides which bind and activate ErbB-3. Examples of ErbB-3 ligands include, but are not limited to neuregulin 1 (NRG) and neuregulin 2, betacellulin, heparin-binding epidermal growth factor, and epiregulin. The term includes biologically active fragments and/or variants of a naturally occurring polypeptide.

Preferably, the ligand-induced receptor function of ErbB-3 is ErbB-3 ligand-induced growth of an ErbB-2 and ErbB-3 positive cell. In a preferred embodiment said cell is an MCF-7 cell (ATCC® HTB-22™); an SKBR3 (ATCC® HTB-30™) cell; an NCI-87 (ATCC® CRL-5822™) cell; a BxPC-3-luc2 cell (Perkin Elmer 125058), a BT-474 cell (ATCC® HTB-20™) or a JIMT-1 cell (DSMZ no.: ACC 589).

As used herein the ligand-induced receptor function is reduced by at least 20%, preferably at least 30, 40, 50 60, or at least 70% in a particularly preferred embodiment the ligand-induced receptor function is reduced by 80, more preferably by 90%. The reduction is preferably determined by determining a ligand-induced receptor function in the presence of a bispecific antibody disclosed herein, and comparing it with the same function in the absence of the antibody, under otherwise identical conditions. The conditions comprise at least the presence of an ErbB-3 ligand. The amount of ligand present is preferably an amount that induces half of the maximum growth of an ErbB-2 and ErbB-3 positive cell line. The ErbB-2 and ErbB-3 positive cell line for this test is preferably the MCF-7 cell line (ATCC® HTB-22™), the SKBR3 cell line (ATCC® HTB-30™) cells, the JIMT-1 cell line (DSMZ ACC 589) or the NCI-87 cell line (ATCC® CRL-5822™). The test and/or the ligand for determining ErbB-3 ligand-induced receptor function is preferably a test for ErbB-3 ligand induced growth reduction as specified in the examples.

The ErbB-2 protein contains several domains (see for reference figure 1 of Landgraf, R Breast Cancer Res. 2007; 9(1): 202-). The extracellular domains are referred to as domains I-IV. The place of binding to the respective domains of antigen-binding sites of antibodies described herein has been mapped. A bispecific antibody with an antigen-binding site (first antigen-binding site) that binds domain I or domain IV of ErbB-2 (first antigen-binding site) comprises a heavy chain variable region that maintains significant binding specificity and affinity for ErbB-2 when combined with various light chains. Bispecific antibodies with an antigen-binding site (first antigen-binding site) that binds domain I or domain IV of ErbB-2 (first antigen-binding site) and an antigen-binding site for ErbB-3 (second antigen-binding site) are more effective in reducing a ligand-induced receptor function of ErbB-3 when compared to a bispecific antibody comprising an antigen-binding site (first antigen-binding site) that binds to another extra-cellular domain of ErbB-2. A bispecific antibody comprising an antigen-binding site (first antigen-binding site) that binds ErbB-2, wherein said antigen-binding site binds to domain I or domain IV of ErbB-2 is preferred. Preferably said antigen-binding site binds to domain IV of ErbB-2. Preferred antibodies comprises a first antigen-binding site that binds domain I of ErbB-2 and a second antigen-binding site that binds domain III of ErbB-3.

In one preferred embodiment, said antibody comprises an antigen-binding site that binds at least one amino acid of domain I of ErbB-2 selected from the group consisting of T144, T164, R166, P172, G179, S180 and R181, and surface-exposed amino acid residues that are located within about 5 amino acid positions from T144, T164, R166, P172, G179, S180 or R181.

In one preferred embodiment, said antibody preferably comprises an antigen-binding site that binds at least one amino acid of domain III of ErbB-3 selected from the group consisting of R426 and surface-exposed amino acid residues that are located within 11.2 Å from R426 in the native ErbB-3 protein.

A bispecific antibody with an antigen-binding site (first antigen-binding site) that binds ErbB-2, and that further comprises ADCC are more effective than other ErbB-2 binding antibodies that did not have significant ADCC activity, particularly *in vivo.* A bispecific antibody which exhibits ADCC is therefore preferred. It was found that antibodies wherein said first antigen-binding site binds to domain IV of ErbB-2 had intrinsic ADCC activity. A domain I binding ErbB-2 binding antibody that has low intrinsic ADCC activity can be engineered to enhance the ADCC activity Fc regions mediate antibody function by binding to different receptors on immune effector cells such as macrophages, natural killer cells, B-cells and neutrophils. Some of these receptors, such as CD16A (FcyRIIIA) and CD32A (FcyRIIA), activate the cells to build a response against antigens. Other receptors, such as CD32B, inhibit the activation of immune cells. By engineering Fc regions (through introducing amino acid substitutions) that bind to activating receptors with greater selectivity, antibodies can be created that have greater capability to mediate cytotoxic activities desired by an anti-cancer Mab.

One technique for enhancing ADCC of an antibody is afucosylation. (See for instance Junttila, T. T., K. Parsons, et al. (2010). "Superior In vivo Efficacy of Afucosylated Trastuzumab in the Treatment of HER2-Amplified Breast Cancer." Cancer Research 70(11): 4481-4489). Further provided is therefore a bispecific antibody as disclosed herein, which is afucosylated. Alternatively, or additionally, multiple other strategies can be used to achieve ADCC enhancement, for instance including glycoengineering (Kyowa Hakko/Biowa, GlycArt (Roche) and Eureka Therapeutics) and mutagenesis (Xencor and Macrogenics), all of which seek to improve Fc binding to low-affinity activating FcyRIIIa, and/or to reduce binding to the low affinity inhibitory FcγRIIb.

Several *in vitro* methods exist for determining the efficacy of antibodies or effector cells in eliciting ADCC. Among these are chromium-51 [Cr51] release assays, europium [Eu] release assays, and sulfur-35 [S35] release assays. Usually, a labeled target cell line expressing a certain surface-exposed antigen is incubated with antibody specific for that antigen. After washing, effector cells expressing Fc receptor CD16 are typically co-incubated with the antibody-labeled target cells. Target cell lysis is subsequently typically measured by release of intracellular label, for instance by a scintillation counter or spectrophotometry.

In preferred bispecific antibodies, the affinity of said second antigen-binding site for an ErbB-3 positive cell is equal to, or preferably higher than, the affinity of said first antigen-binding site for an ErbB-2 positive cell. The affinity (KD) of said second antigen-binding site for an ErbB-3 positive cell is preferably lower than or equal to 2.0 nM, more preferably lower than or equal to 1.5 nM, more preferably lower than or equal to 1.39 nM, more preferably lower than or equal to 0.99 nM. In one preferred embodiment, the affinity of said second antigen-binding site for ErbB-3 on SK-BR-3 cells is lower than or equal to 2.0 nM, more preferably lower than or equal to 1.5 nM, more preferably lower than or equal to 1.39 nM, preferably lower than or equal to 0.99 nM. In one embodiment, said affinity is within the range of 1.39-0.59 nM. In one preferred embodiment, the affinity of said second antigen-binding site for ErbB-3 on BT-474 cells is lower than or equal to 2.0 nM, more preferably lower than or equal to 1.5 nM, more preferably lower than or equal to 1.0 nM, more preferably lower than 0.5 nM, more preferably lower than or equal to 0.31 nM, more preferably lower than or equal to 0.23 nM. In one embodiment, said affinity is within the range of 0.31-0.15 nM. The above-mentioned affinities are preferably as measured using steady state cell affinity measurements, wherein cells are incubated at 4°C using radioactively labeled antibody, where after cell-bound radioactivity is measured, as described in the Examples of WO 2015/130173.

The affinity (KD) of said first antigen-binding site for an ErbB-2 positive cell is preferably lower than or equal to 5.0 nM, more preferably lower than or equal to 4.5 nM, more preferably lower than or equal to 3.9 nM. In one preferred embodiment, the affinity of said first antigen-binding site for ErbB-2 on SK-BR-3 cells is lower than or equal to 5.0 nM, preferably lower than or equal to 4.5 nM, more preferably lower than or equal to 4.0 nM, more preferably lower than or equal to 3.5 nM, more preferably lower than or equal to 3.0 nM, more preferably lower than or equal to 2.3 nM. In one embodiment, said affinity is within the range of 3.0-1.6 nM. In one preferred embodiment, the affinity of said first antigen-binding site for ErbB-2 on BT-474 cells is lower than or equal to 5.0 nM, preferably lower than or equal to 4.5 nM, more preferably lower than or equal to 3.9 nM. In one embodiment, said affinity is within the range of 4.5-3.3 nM. The above-mentioned affinities are preferably as measured using steady state cell affinity measurements, wherein cells are incubated at 4°C using radioactively labeled antibody, where after cell-bound radioactivity is measured, as described in the Examples of WO 2015/130173.

Preferably, the bispecific antibodies used in the disclosed methods do not significantly affect the survival of cardiomyocytes. Cardiotoxicity is a known risk factor in ErbB-2 targeting therapies and the frequency of complications is increased when trastuzumab is used in conjunction with anthracyclines thereby inducing cardiac stress.

The bispecific antibodies disclosed herein are preferably used in humans. thus, preferred antibodies are human or humanized antibodies. Tolerance of a human to a polypeptide is governed by many different aspects. Immunity, be it T-cell mediated, B-cell mediated or other is one of the variables that are encompassed in tolerance of the human for a polypeptide. The constant region of a bispecific antibody is preferably a human constant region. The constant region may contain one or more, preferably not more than 10, preferably not more than 5 amino-acid differences with the constant region of a naturally occurring human antibody. It is preferred that the constant part is entirely derived from a naturally occurring human antibody. Various antibodies produced herein are derived from a human antibody variable domain library. As such these variable domains are human. The unique CDR regions may be derived from humans, be synthetic or derived from another organism. The variable region is considered a human variable region when it has an amino acid sequence that is identical to an amino acid sequence of the variable region of a naturally occurring human antibody, but for the CDR region. The variable region of an ErbB-2 binding VH, an ErbB-3 binding VH, or a light chain in an antibody may contain one or more, preferably not more than 10, preferably not more than 5 amino-acid differences with the variable region of a naturally occurring human antibody, not counting possible differences in the amino acid sequence of the CDR regions. Such mutations occur also in nature in the context of somatic hypermutation.

Antibodies may be derived from various animal species, at least with regard to the heavy chain variable region. It is common practice to humanize such e.g. murine heavy chain variable regions. There are various ways in which this can be achieved among which there are CDR-grafting into a human heavy chain variable region with a 3D-structure that matches the 3-D structure of the murine heavy chain variable region; deimmunization of the murine heavy chain variable region, preferably done by removing known or suspected T- or B- cell epitopes from the murine heavy chain variable region. The removal is typically by substituting one or more of the amino acids in the epitope for another (typically conservative) amino acid, such that the sequence of the epitope is modified such that it is no longer a Tor B-cell epitope.

Such deimmunized murine heavy chain variable regions are less immunogenic in humans than the original murine heavy chain variable region. Preferably a variable region or domain is further humanized, such as for instance veneered. By using veneering techniques, exterior residues which are readily encountered by the immune system are selectively replaced with human residues to provide a hybrid molecule that comprises either a weakly immunogenic or substantially non-immunogenic veneered surface. An animal as used in the invention is preferably a mammal, more preferably a primate, most preferably a human.

A bispecific antibody disclosed herein preferably comprises a constant region of a human antibody. According to differences in their heavy chain constant domains, antibodies are grouped into five classes, or isotypes: IgG, IgA, IgM, IgD, and IgE. These classes or isotypes comprise at least one of said heavy chains that is named with a corresponding Greek letter. Preferably the constant region comprises an IgG constant region, more preferably an IgG1 constant region, preferably a mutated IgG1 constant region. Some variation in the constant region of IgG1 occurs in nature, such as for instance the allotypes G1m1, 17 and G1m3, and/or is allowed without changing the immunological properties of the resulting antibody. Typically between about 1-10 amino acid insertions, deletions, substitutions or a combination thereof are allowed in the constant region.

Preferred bispecific antibodies as disclosed herein comprise:

- at least the CDR3 sequence, preferably at least the CDR1, CDR2 and CDR3 sequences, or at least the heavy chain variable region sequence, of an ErbB-2 specific heavy chain variable region selected from the group consisting of MF2926, MF2930, MF1849; MF2973, MF3004, MF3958, MF2971, MF3025, MF2916, MF3991, MF3031, MF2889, MF2913, MF1847, MF3001, MF3003 and MF1898, or a heavy chain variable region sequence that differs in at most 15 amino acids, preferably in at most 1, 2, 3, 4, 5, 6, 7, 8, 9 or 10 amino acids, more preferably in at most 1, 2, 3, 4 or 5 amino acids, from the recited heavy chain variable region sequences; and/or

- at least the CDR3 sequence, preferably at least the CDR1, CDR2 and CDR3 sequences, or at least the heavy chain variable region sequence, of an ErbB-3 specific heavy chain variable region selected from the group consisting of MF3178; MF3176; MF3163; MF3099; MF3307; MF6055; MF6056; MF6057; MF6058; MF6059; MF6060; MF6061; MF6062; MF6063; MF6064; MF 6065; MF6066; MF6067; MF6068; MF6069; MF6070; MF6071; MF6072; MF6073 and MF6074, or a heavy chain variable region sequence that differs in at most 15 amino acids, preferably in at most 1, 2, 3, 4, 5, 6, 7, 8, 9 or 10 amino acids, more preferably in at most 1, 2, 3, 4 or 5 amino acids, from the recited heavy chain variable region sequences.

CDR sequences are for instance varied for optimization purposes, preferably in order to improve binding efficacy or the stability of the antibody. Optimization is for instance performed by mutagenesis procedures where after the stability and/or binding affinity of the resulting antibodies are preferably tested and an improved ErbB-2 or ErbB-3 -specific CDR sequence is preferably selected. A skilled person is well capable of generating antibody variants comprising at least one altered CDR sequence. For instance, conservative amino acid substitution is applied. Examples of conservative amino acid substitution include the substitution of one hydrophobic residue such as isoleucine, valine, leucine or methionine for another hydrophobic residue, and the substitution of one polar residue for another polar residue, such as the substitution of arginine for lysine, glutamic acid for aspartic acid, or glutamine for asparagine.

Preferred antibodies comprise a variable domain that binds ErbB-2, wherein the VH chain of said variable domain comprises the amino acid sequence of VH chain MF2926; MF2930; MF1849; MF2973; MF3004; MF3958 (is humanized MF2971); MF2971; MF3025; MF2916; MF3991 (is humanized MF3004); MF3031; MF2889; MF2913; MF1847; MF3001, MF3003 or MF1898; or comprises the amino acid sequence of VH chain MF2926; MF2930; MF1849; MF2973; MF3004; MF3958 (is humanized MF2971); MF2971; MF3025; MF2916; MF3991 (is humanized MF3004); MF3031; MF2889; MF2913; MF1847; MF3001, MF3003 or MF1898 as having at most 15, preferably 1, 2, 3, 4, 5, 6, 7, 8, 9 or 10 more preferably at most 1, 2, 3, 4 or 5, amino acid insertions, deletions, substitutions or a combination thereof with respect to the above mentioned VH chain sequence. The VH chain of the variable domain that binds ErbB-2 preferably comprises the amino acid sequence of:

- MF1849; or
- MF2971 or a humanized version thereof, wherein said humanized version preferably comprises the amino acid sequence of MF3958; or
- MF3004 or a humanized version thereof, wherein said humanized version preferably comprises the amino acid sequence of MF3991. In one embodiment, the VH chain of the variable domain that binds ErbB-2 comprises the amino acid sequence of VH chain MF1849; or MF2971 or a humanized version thereof, wherein said humanized version preferably comprises the amino acid sequence of MF3958; or MF3004 or a humanized version thereof, wherein said humanized version preferably comprises the amino acid sequence of MF3991, wherein the recited VH sequences have at most 15, preferably 1, 2, 3, 4, 5, 6, 7, 8, 9 or 10, more preferably at most 1, 2, 3, 4 or 5, amino acid insertions, deletions, substitutions or a combination thereof with respect to the respective sequence. In a preferred embodiment the VH chain of the variable domain that binds ErbB-2 comprises the amino acid sequence of MF3958; or comprises the amino acid sequence of MF3958 having at most 15, preferably 1, 2, 3, 4, 5, 6, 7, 8, 9 or 10, more preferably at most 1, 2, 3, 4 or 5, amino acid insertions, deletions, substitutions or a combination thereof with respect to the VH chain sequence.

The VH chain of the variable domain that binds Erb-B3 preferably comprises the amino acid sequence of VH chain MF3178; MF3176; MF3163; MF3099; MF3307; MF6055; MF6056; MF6057; MF6058; MF6059; MF6060; MF6061; MF6062; MF6063; MF6064; MF 6065; MF6066; MF6067; MF6068; MF6069; MF6070; MF6071; MF6072; MF6073 or MF6074; or comprises the amino acid sequence of VH chain MF3178; MF3176; MF3163; MF3099; MF3307; MF6055; MF6056; MF6057; MF6058; MF6059; MF6060; MF6061; MF6062; MF6063; MF6064; MF 6065; MF6066; MF6067; MF6068; MF6069; MF6070; MF6071; MF6072; MF6073 or MF6074 having at most 15, preferably 1, 2, 3, 4, 5, 6, 7, 8, 9 or 10, more preferably at most 1, 2, 3, 4 or 5, amino acid insertions, deletions, substitutions or a combination thereof with respect to the VH chain sequence. The VH chain of the variable domain that binds Erb-B3 preferably comprises the amino acid sequence of MF3178, MF3176, MF3163, MF6058, MF6061 or MF6065; or comprises the amino acid sequence of MF3178, MF3176, MF3163, MF6058, MF6061 or MF6065 having at most 15, preferably 1, 2, 3, 4, 5, 6, 7, 8, 9 or 10, more preferably in at most 1, 2, 3, 4 or 5, amino acid insertions, deletions, substitutions or a combination thereof with respect to the respective VH chain sequence. In a preferred embodiment the VH chain of the variable domain that binds ErbB-3 comprises the amino acid sequence of MF3178; or comprises the amino acid sequence of MF3178 having at most 15, preferably 1, 2, 3, 4, 5, 6, 7, 8, 9 or 10, more preferably at most 1, 2, 3, 4 or 5, amino acid insertions, deletions, substitutions or a combination thereof with respect to the VH chain sequence. Preferably, the above-mentioned amino acid insertions, deletions and substitutions are not present in the CDR3 region. The above-mentioned amino acid insertions, deletions and substitutions are also preferably not present in the CDR1 and CDR2 regions. The above-mentioned amino acid insertions, deletions and substitutions are also preferably not present in the FR4 region.

Preferably, the antibody comprises at least the CDR1, CDR2 and CDR3 sequences of MF1849, MF2971, MF3958, MF3004 or MF3991, most preferably at least the CDR1, CDR2 and CDR3 sequences of MF3958. Said antibody preferably comprises at least the CDR1, CDR2 and CDR3 sequences of MF3178, MF3176, MF3163, MF6058, MF6061 or MF6065, most preferably at least the CDR1, CDR2 and CDR3 sequence of MF3178.

Preferably, the ErbB-2 specific heavy chain variable region comprises the amino acid sequence of the VH chain MF3958 having at most 15, preferably 1, 2, 3, 4, 5, 6, 7, 8, 9 or 10, more preferably at most 1, 2, 3, 4 or 5, amino acid insertions, deletions, substitutions or a combination thereof with respect said VH (preferably wherein said insertions, deletions, substitutions are not in CDR1, CDR2, or CDR3). Preferably, the ErbB-3 specific heavy chain variable region comprises the amino acid sequence of the VH chain MF3178 having at most 15, preferably 1, 2, 3, 4, 5, 6, 7, 8, 9 or 10, more preferably at most 1, 2, 3, 4 or 5, amino acid insertions, deletions, substitutions or a combination thereof with respect said VH. The one or more amino acid insertions, deletions, substitutions or a combination thereof are preferably not in the CDR1, CDR2 and CDR3 region of the VH chain. They are also preferably not present in the FR4 region. An amino acid substitution is preferably a conservative amino acid substitution.

Preferably, the ErbB-2 specific heavy chain variable region comprises the amino acid sequence of the VH chain MF3991 having at most 15, preferably 1, 2, 3, 4, 5, 6, 7, 8, 9 or 10, more preferably at most 1, 2, 3, 4 or 5, amino acid insertions, deletions, substitutions or a combination thereof with respect said VH (preferably wherein said insertions, deletions, substitutions are not in CDR1, CDR2, or CDR3). Preferably, the ErbB-3 specific heavy chain variable region comprises the amino acid sequence of the VH chain MF3178 having at most 15, preferably 1, 2, 3, 4, 5, 6, 7, 8, 9 or 10, more preferably at most 1, 2, 3, 4 or 5, amino acid insertions, deletions, substitutions or a combination thereof with respect said VH. The one or more amino acid insertions, deletions, substitutions or a combination thereof are preferably not in the CDR1, CDR2 and CDR3 region of the VH chain. They are also preferably not present in the FR4 region. An amino acid substitution is preferably a conservative amino acid substitution.

Preferably, the first antigen-binding site of the antibody comprises at least the CDR1, CDR2 and CDR3 sequences of MF3958, or CDR1, CDR2 and CDR3 sequences that differ in at most three, preferably in at most two, preferably in at most one amino acid from the CDR1, CDR2 and CDR3 sequences of MF3958, and wherein said second antigen-binding site comprises at least the CDR1, CDR2 and CDR3 sequence of MF3178, or CDR1, CDR2 and CDR3 sequences that differ in at most three, preferably in at most two, preferably in at most one amino acid from the CDR1, CDR2 and CDR3 sequences of MF3178.

Preferably, the bispecific antibody comprises i) a first antigen binding site comprising an ErbB-2 specific heavy chain variable region comprising the CDR1, CDR2, and CDR3 sequence of MF3958 and a light chain variable region and ii) a second antigen binding site comprising an ErbB-3 specific heavy chain variable region comprising the CDR1, CDR2, and CDR3 sequence of MF3178 and a light chain variable region.

Preferably, the ErbB-2 specific heavy chain variable region has the MF3958 sequence and the ErbB-3 specific heavy chain variable region has the MF3178 sequence. This combination is also referred to as the PB4188 antibody. Preferably, the PB4188 antibody is afucosylated.

Preferably, the the bispecific antibody comprises the "heavy cahin for erbB-2 binding" as depicted in the Sequence listing part 1D and the "heavy cahin for erbB-3 binding" as depicted in the Sequence listing part 1D.

Preferably, the antigen binding sites of the bispecific antibody comprise a germline light chain O12, preferably the rearranged germline human kappa light chain IgVκ1-39*01/IGJκ1*01 or a fragment or a functional derivative thereof (nomenclature according to the IMGT database worldwide web at imgt.org). The terms rearranged germline human kappa light chain IgVκ1-39*01/IGJκ1*01, IGKV1-39/IGKJ1, huVκ1-39 light chain or in short huVκ1-39 are used. The light chain can have 1, 2, 3, 4 or 5 amino acid insertions, deletions, substitutions or a combination thereof. The mentioned 1, 2, 3, 4 or 5 amino acid substitutions are preferably conservative amino acid substitutions, the insertions, deletions, substitutions or a combination thereof are preferably not in the CDR3 region of the VL chain, preferably not in the CDR1, CDR2 or CDR3 region or FR4 region of the VL chain. Preferably, the first antigen binding site and the second antigen binding site comprise the same light chain variable region, or rather, a common light chain. Preferably, the light chain variable region comprises a CDR1 having the sequence (RASQSISSYLN), a CDR2 having the sequence (AASSLQS), and a CDR3 having the sequence (QQSYSTPPT). Preferably, the light chain variable region comprises the common light chain sequence depicted the Sequence listing part 1C.

Various methods are available to produce bispecific antibodies and are discussed in WO 2015/130173. One method involves the expression of two different heavy chains and two different light chains in a cell and collecting antibody that is produced by the cell. Antibody produced in this way will typically contain a collection of antibodies with different combinations of heavy and light chains, some of which are the desired bispecific antibody. The bispecific antibody can subsequently be purified from the collection.

The ratio of bispecific to other antibodies that are produced by the cell can be increased in various ways.

Preferably, the ratio is increased by expressing not two different light chains but two essentially identical light chains in the cell. This concept is in the art also referred to as the "common light chain" method. When the essentially identically light chains work together with the two different heavy chains allowing the formation of variable domains with different antigen-binding sites and concomitant different binding properties, the ratio of bispecific antibody to other antibody that is produced by the cell is significantly improved over the expression of two different light chains. The ratio of bispecific antibody that is produced by the cell can be further improved by stimulating the pairing of two different heavy chains with each other over the pairing of two identical heavy chains. The art describes various ways in which such heterodimerization of heavy chains can be achieved. One way is to generate 'knob into hole' bispecific antibodies. See US Patent Application 20030078385 (Arathoon et al. - Genentech). Another and preferred method is described in PCT application No. PCT/NL2013/050294 (WO 2013/157954 A1), which aisre incorporated herein by reference. Methods and means are disclosed for producing bispecific antibodies from a single cell, whereby means are provided that favor the formation of bispecific antibodies over the formation of monospecific antibodies.

Sequences referred to in the disclosure are presented below and in Figure 1.

**Sequences 1A (erbB-2 specific)**

**MF2926:** heavy chain variable region sequence of an erbB-2 binding antibody Nucleic acid sequence (underlined sequence encodes end of leader peptide):

```
1   GGCCCAGCCG GCCATGGCCC AGGTCCAGCT GCAGCAGTCT GGACCTGAGC TGGTGAAACC
61  TGGGGCTTCA GTGATGATTT CCTGCAAGGC TTCTGGTTAC TCATTCACTG GCTACCACAT
121 GAACTGGGTG AAGCAAAGTC CTGAAAAGAG CCTTGAGTGG ATTGGAGACA TAAATCCTAG
181 CATTGGTACG ACTGCCCACA ACCAGATTTT CAGGGCCAAG GCCACAATGA CTGTTGACAA
241 ATCCTCCAAC ACAGCCTACA TGCAGCTCAA GAGCCTGACA TCTGAAGACT CTGGAGTCTT
301 TTACTGTGTT AGAAGAGGGG ACTGGTCCTT CGATGTCTGG GGCACAGGGA CCACGGTCAC
361 CGTCTCCAGT
```

Amino acid sequence:

QVQLQQSGPELVKPGASVMISCKASGYSFTGYHMNWVKQSPEKSLEWIGDINP
SIGTTAHNQIFRAKATMTVDKSSNTAYMQLKSLTSEDSGVFYCVRRGDWSFDV
WGTGTTVTVSS

| | |
|---|---|
| CDR1: | GYHMNWVKQSPEKSLE |
| CDR2: | NQIFRA |
| CDR3: | RGDWSFDV |

**MF2930:** heavy chain variable region sequence of an erbB-2 binding antibody Nucleic acid sequence (underlined sequence encodes end of leader peptide):

1 GGCCCAGCCG GCCATGGCCG AGGTCCAGCT GCAGCAGTCT GGGGCTGAAC TGGTGAAGCC
61 TGGAGCCTCA GTGATGATGT CCTGTAAGGT TTCTGGCTAC ACCTTCACTT CCTATCCTAT
121 AGCGTGGATG AAGCAGGTTC ATGGAAAGAG CCTAGAGTGG ATTGGAAATT TTCATCCTTA
181 CAGTGATGAT ACTAAGTACA ATGAAAACTT CAAGGGCAAG GCCACATTGA CTGTAGAAAA
241 ATCCTCTAGC ACAGTCTACT TGGAGCTCAG CCGATTAACA TCTGATGACT CTGCTGTTTA
301 TTACTGTGCA AGAAGTAACC CATTATATTA CTTTGCTATG GACTACTGGG GTCAAGGAAC
361 CTCGGTCACC GTCTCCAGT

Amino acid sequence:

EVQLQQSGAELVKPGASVMMSCKVSGYTFTSYPIAWMKQVHGKSLEWIGNFH PYSDDTKYNENFKGKATLTVEKSSSTVYLELSRLTSDDSAVYYCARSNPLYYFA MDYWGQGTSVTVSS

| | |
|---|---|
| CDR1: | SYPIAWMKQVHGKSLE |
| CDR2: | NENFKG |
| CDR3: | SNPLYYFAMDY |

**MF1849:** heavy chain variable region sequence of an erbB-2 binding antibody Nucleic acid sequence (underlined sequence encodes end of leader peptide):

1 GGCCCAGCCG GCCATGGCCC AGGTGCAGCT GGTGGAGTCT GGGGGAGGCG TGGTCCAGCC
61 TGGGAGGTCC CTGAGACTCT CCTGTGCAGC CTCTGGATTC ACCTTCAGTA GCTATGGCAT
121 GCACTGGGTC CGCCAGGCTC CAGGCAAGGG GCTGGAGTGG GTGGCAGTTA TATCATATGA
181 TGGAAGTAAT AAATACTATG CAGACTCCGT GAAGGGCCGA TTCACCATCT CCAGAGACAA
241 TTCCAAGAAC ACGCTGTATC TGCAAATGAA CAGCCTGAGA GCTGAGGACA CGGCCGTGTA
301 TTACTGTGCA AAAGGTGACT ACGGTTCTTA CTCTTCTTAC GCCTTTGATT ATTGGGGCCA
361 AGGTACCCTG GTCACCGTCT CCAGT

Amino acid sequence:

QVQLVESGGGVVQPGRSLRLSCAASGFTFSSYGMHWVRQAPGKGLEWVAVISY
DGSNKYYADSVKGRFTISRDNSKNTLYLQMNSLRAEDTAVYYCAKGDYGSYSS
YAFDYWGQGTLVTVSS

CDR1: SYGMH
CDR2: VISYDGSNKYYADSVKG
CDR3: GDYGSYSSYAFDY

**MF2973:** heavy chain variable region sequence of an erbB-2 binding antibody Nucleic acid sequence (underlined sequence encodes end of leader peptide):

```
1 GGCCCAGCCG GCCATGGCCC AGGTGCAGCT GAAGCAGTCT GGGGCTGAGC
TGGTGAGGCC
61 TGGGGCTTCA GTGAAGTTGT CCTGCAAGGC TTCTGGCTAC ATTTTCACTG
GCTACTATAT
121 AAACTGGTTG AGGCAGAGGC CTGGACAGGG ACTTGAATGG ATTGCAAAAA
TTTATCCTGG
181 AAGTGGTAAT ACTTACTACA ATGAGAAGTT CAGGGGCAAG GCCACACTGA
CTGCAGAAGA
241 ATCCTCCAGC ACTGCCTACA TGCAGCTCAG CAGCCTGACA TCTGAGGACT
CTGCTGTCTA
301 TTTCTGTGCA AGAGGGCCCC ACTATGATTA CGACGGCCCC TGGTTTGTTT
ACTGGGGCCA
361 AGGGACTCTG GTCACCGTCT CCAGT
```

Amino acid sequence:

QVQLKQSGAELVRPGASVKLSCKASGYIFTGYYINWLRQRPGQGLEWIAKIYPG
SGNTYYNEKFRGKATLTAEESSSTAYMQLSSLTSEDSAVYFCARGPHYDYDGP
WFVYWGQGTLVTVSS

CDR1: GYYINWLRQRPGQGLE
CDR2: NEKFRG
CDR3: GPHYDYDGPWFVY

**MF3004:** heavy chain variable region sequence of an erbB-2 binding antibody Nucleic acid sequence (underlined sequence encodes end of leader peptide):

1 GGCCCAGCCG GCCATGGCCC AGGTGCAGCT GAAGCAGTCT GGGGCTGAGC TGGTGAGGCC

61 TGGGGCTTCA GTGAAGCTGT CCTGCAAGGC TTCTGGCTAC ACTTTCACTG GCTACTATAT

121 AAACTGGGTG AAGCAGAGGC CTGGACAGGG ACTTGAGTGG ATTGCAAGGA TTTATCCTGG

181 AAGTGGTTAT ACTTACTACA ATGAGAAGTT CAAGGGCAAG GCCACACTGA CTGCAGAAGA

241 ATCCTCCAGC ACTGCCTACA TGCACCTCAG CAGCCTGACA TCTGAGGACT CTGCTGTCTA

301 TTTCTGTGCA AGACCCCACT ATGGTTACGA CGACTGGTAC TTCGGTGTCT GGGGCACAGG

361 CACCACGGTC ACCGTCTCCA GT

Amino acid sequence:

QVQLKQSGAELVRPGASVKLSCKASGYTFTGYYINWVKQRPGQGLEWIARIYPG SGYTYYNEKFKGKATLTAEESSSTAYMHLSSLTSEDSAVYFCARPHYGYDDWY FGVWGTGTTVTVSS

CDR1: GYYINWVKQRPGQGLE
CDR2: NEKFKG
CDR3: PHYGYDDWYFGV

**MF2971:** heavy chain variable region sequence of an erbB-2 binding antibody Nucleic acid sequence (underlined sequence encodes end of leader peptide):

1 GGCCCAGCCG GCCATGGCCC AGGTGCAGCT GAAGCAGTCT GGGGCTGAGC TGGTGAGGCC

61 TGGGGCTTCA GTGAAACTGT CCTGCAAGGC TTCTGGCTAC ACTTTCACTG CCTACTATAT

121 AAACTGGGTG AAGCAGAGGC CTGGACAGGG ACTTGAGTGG ATTGCAAGGA TTTATCCTGG

181 AAGTGGCTAT ACTTACTACA ATGAGATTTT CAAGGGCAGG GCCACACTGA CTGCAGACGA

241 ATCCTCCAGC ACTGCCTACA TGCAACTCAG CAGCCTGACA TCTGAGGACT CTGCTGTCTA

301 TTTCTGTGCA AGACCTCCGG TCTACTATGA CTCGGCCTGG TTTGCTTACT GGGGCCAAGG

361 GACTCTGGTC ACCGTCTCCA GT

Amino acid sequence:

QVQLKQSGAELVRPGASVKLSCKASGYTFTAYYINWVKQRPGQGLEWIARIYPG
SGYTYYNEIFKGRATLTADESSSTAYMQLSSLTSEDSAVYFCARPPVYYDSAWF
AYWGQGTLVTVSS

CDR1: AYYINWVKQRPGQGLE
CDR2: NEIFKG
CDR3: PPVYYDSAWFAY

**MF3025:** heavy chain variable region sequence of an erbB-2 binding antibody Nucleic acid sequence (underlined sequence encodes end of leader peptide):

```
1 GGCCCAGCCG GCCATGGCCC AGGTGCAGCT GAAGCAGTCT GGGGCTGAGC TGGTGAGGCC
61 TGGGACTTCA GTGAAGCTGT CCTGCAAGGC TTCTGGCTAC ACTTTCACTG GCTACTATAT
121 AAACTGGGTG AAGCAGAGGC CTGGACAGGG ACTTGAGTGG ATTGCAAGGA TTTATCCTGG
181 AAGTGGTTAT ACTTACTACA ATGAGAAGTT CAAGGGCAAG GCCACACTGA CTGCAGAAGA
241 ATCCTCCAAC ACTGCCTATA TGCACCTCAG CAGCCTGACA TCTGAGGACT CTGCTGTCTA
301 TTTCTGTGCA AGGCCCCACT ATGGTTACGA CGACTGGTAC TTCGCTGTCT GGGGCACAGG
361 GACCACGGTC ACCGTCTCCA GT
```

Amino acid sequence:

QVQLKQSGAELVRPGTSVKLSCKASGYTFTGYYINWVKQRPGQGLEWIARIYPG
SGYTYYNEKFKGKATLTAEESSNTAYMHLSSLTSEDSAVYFCARPHYGYDDWY
FAVWGTGTTVTVSS

CDR1: GYYINWVKQRPGQGLE
CDR2: NEKFKG
CDR3: PHYGYDDWYFAV

**MF2916:** heavy chain variable region sequence of an erbB-2 binding antibody Nucleic acid sequence (underlined sequence encodes end of leader peptide):

1 GGCCCAGCCG GCCATGGCCC AGGTCCAGCT GCAGCAGTCT GGGGCTGAGC TGGTGAGGCC

61 TGGGGCTTCA GTGAAGCTGT CCTGCAAGGC TTCTGGCTAC ACTTTCACTG GCTACTATAT

121 AAACTGGGTG AAGCAGAGGC CTGGACAGGG ACTTGAGTGG ATTGCAAGGA TTTATCCTGG

181 CAGTGGTCAT ACTTCCTACA ATGAGAAGTT CAAGGGCAAG GCCACACTGA CTACAGAAAA

241 ATCCTCCAGC ACTGCCTACA TGCAGCTCAG CAGCCTGACA TCTGAGGACT CTGCTGTCTA

301 TTTCTGTGCA AGACCTATCT ACTTTGATTA CGCAGGGGGG TACTTCGATG TCTGGGGCAC

361 AAGAACCTCG GTCACCGTCT CCAGT

Amino acid sequence:

QVQLQQSGAELVRPGASVKLSCKASGYTFTGYYINWVKQRPGQGLEWIARIYPG SGHTSYNEKFKGKATLTTEKSSSTAYMQLSSLTSEDSAVYFCARPIYFDYAGGY FDVWGTRTSVTVSS

| | |
|---|---|
| CDR1: | GYYINWVKQRPGQGLE |
| CDR2: | NEKFKG |
| CDR3: | PIYFDYAGGYFDV |

**MF3958:** heavy chain variable region sequence of an erbB-2 binding antibody Nucleic acid sequence (underlined sequence encodes end of leader peptide):

1 GGCCCAGCCG GCCATGGCCC AGGTGCAGCT GGTGCAGTCT GGCGCCGAAG TGAAGAAACC

61 TGGCGCCAGC GTGAAGCTGA GCTGCAAGGC CAGCGGCTAC ACCTTCACCG CCTACTACAT

121 CAACTGGGTC CGACAGGCCC CAGGCCAGGG CCTGGAATGG ATCGGCAGAA TCTACCCCGG

181 CTCCGGCTAC ACCAGCTACG CCCAGAAGTT CCAGGGCAGA GCCACCCTGA CCGCCGACGA

241 GAGCACCAGC ACCGCCTACA TGGAACTGAG CAGCCTGCGG AGCGAGGATA CCGCCGTGTA

301 CTTCTGCGCC AGACCCCCCG TGTACTACGA CAGCGCTTGG TTTGCCTACT GGGGCCAGGG

361 CACCCTGGTC ACCGTCTCCA GT

Amino acid sequence:

QVQLVQSGAEVKKPGASVKLSCKASGYTFTAYYINWVRQAPGQGLEWIGRIYPG
SGYTSYAQKFQGRATLTADESTSTAYMELSSLRSEDTAVYFCARPPVYYDSAWF
AYWGQGTLVTVSS

CDR1: AYYIN
CDR2: RIYPGSGYTSYAQKFQG
CDR3: PPVYYDSAWFAY

**MF3031:** heavy chain variable region sequence of an erbB-2 binding antibody Nucleic acid sequence (underlined sequence encodes end of leader peptide):

```
1 GGCCCAGCCG GCCATGGCCC AGGTCCAGCT GCAGCAGTCT GGGGCTGAGC
TGGTGAGGCC
61 TGGGGCTTCA GTGAAGCTGT CCTGCAAGGC TTCTGGCTAC ACTTTCACTG
CCTACTATAT
121 AAACTGGGTG AAGCAGAGGC CTGGACAGGG ACTTGAGTGG ATTGCAAAGA
TTTATCCTGG
181 AAGTGGTTAT ACTTACTACA ATGAGAATTT CAGGGGCAAG GCCACACTGA
CTGCAGAAGA
241 ATCCTCCAGT ACTGCCTACA TACAACTCAG CAGCCTGACA TCTGAGGACT
CTGCTGTCTA
301 TTTCTGTGCA AGAGGCGTCT ATGATTACGA CGGGGCCTGG TTTGCTTACT
GGGGCCAAGG
361 GACTCTGGTC ACCGTCTCCA GT
```

Amino acid sequence:

QVQLQQSGAELVRPGASVKLSCKASGYTFTAYYINWVKQRPGQGLEWIAKIYPG
SGYTYYNENFRGKATLTAEESSSTAYIQLSSLTSEDSAVYFCARGVYDYDGAWF
AYWGQGTLVTVSS

CDR1: AYYINWVKQRPGQGLE
CDR2: NENFRG
CDR3: GVYDYDGAWFAY

**MF3991:** heavy chain variable region sequence of an erbB-2 binding antibody Nucleic acid sequence (underlined sequence encodes end of leader peptide):

1 GGCCCAGCCG GCCATGGCCC AGGTGCAGCT GGTGCAGTCT GGCGCCGAAG TGAAGAAACC

61 TGGCGCCAGC GTGAAGCTGA GCTGCAAGGC CAGCGGCTAC ACCTTCACCG CCTACTACAT

121 CAACTGGGTC CGACAGGCCC CAGGCCAGGG CCTGGAATGG ATCGGCAGAA TCTACCCCGG

181 CTCCGGCTAC ACCAGCTACG CCCAGAAGTT CCAGGGCAGA GCCACCCTGA CCGCCGACGA

241 GAGCACCAGC ACCGCCTACA TGGAACTGAG CAGCCTGCGG AGCGAGGATA CCGCCGTGTA

301 CTTCTGCGCC AGACCCCACT ACGGCTACGA CGACTGGTAC TTCGGCGTGT GGGGCCAGGG

361 CACCCTGGTC ACCGTCTCCA GT

Amino acid sequence:

QVQLVQSGAEVKKPGASVKLSCKASGYTFTAYYINWVRQAPGQGLEWIGRIYPG SGYTSYAQKFQGRATLTADESTSTAYMELSSLRSEDTAVYFCARPHYGYDDWY FGVWGQGTLVTVSS

| | |
|---|---|
| CDR1: | AYYIN |
| CDR2: | RIYPGSGYTSYAQKFQG |
| CDR3: | PHYGYDDWYFGV |

**Sequences 1B** (erbB-3 specific)

MF3178: heavy chain variable region sequence of an erbB-3 binding antibody Nucleic acid sequence (underlined sequence encodes end of leader peptide):

1 <u>GGCCCAGCCG GCCATGGCC</u>C AGGTGCAGCT GGTGCAGTCT GGGGCTGAGG TGAAGAAGCC

61 TGGGGCCTCA GTGAAGGTCT CCTGCAAGGC TTCTGGATAC ACCTTCACCG GCTACTATAT

121 GCACTGGGTG CGACAGGCCC CTGGACAAGG GCTTGAGTGG ATGGGATGGA TCAACCCTAA

181 CAGTGGTGGC ACAAACTATG CACAGAAGTT TCAGGGCAGG GTCACGATGA CCAGGGACAC

241 GTCCATCAGC ACAGCCTACA TGGAGCTGAG CAGGCTGAGA TCTGACGACA CGGCTGTGTA

301 TTACTGTGCA AGAGATCATG GTTCTCGTCA TTTCTGGTCT TACTGGGGCT TTGATTATTG

361 GGGCCAAGGT ACCCTGGTCA CCGTCTCCAG T

Amino acid sequence:

QVQLVQSGAEVKKPGASVKVSCKASGYTFTGYYMHWVRQAPGQGLEWMGWI
NPNSGGTNYAQKFQGRVTMTRDTSISTAYMELSRLRSDDTAVYYCARDHGSRH
FWSYWGFDYWGQGTLVTVSS

CDR1: GYYMH
CDR2: WINPNSGGTNYAQKFQG
CDR3: DHGSRHFWSYWGFDY

**MF3176:** heavy chain variable region sequence of an erbB-3 binding antibody Nucleic acid sequence (underlined sequence encodes end of leader peptide):

```
1 GGCCCAGCCG GCCATGGCCG AGGTGCAGCT GTTGGAGTCT GGGGGAGGCT
TGGTACAGCC
61 TGGGGGGTCC CTGAGACTCT CCTGTGCAGC CTCTGGATTC ACCTTTAGCA
GCTATGCCAT
121 GAGCTGGGTC CGCCAGGCTC CAGGGAAGGG GCTGGAGTGG GTCTCAGCTA
TTAGTGGTAG
181 TGGTGGTAGC ACATACTACG CAGACTCCGT GAAGGGCCGG TTCACCATCT
CCAGAGACAA
241 TTCCAAGAAC ACGCTGTATC TGCAAATGAA CAGCCTGAGA GCCGAGGACA
CGGCTGTGTA
301 TTACTGTGCA AGAGATTGGT GGTACCCGCC GTACTACTGG GGCTTTGATT
ATTGGGGCCA
361 AGGTACCCTG GTCACCGTCT CCAGT
```

Amino acid sequence:

EVQLLESGGGLVQPGGSLRLSCAASGFTFSSYAMSWVRQAPGKGLEWVSAISGS
GGSTYYADSVKGRFTISRDNSKNTLYLQMNSLRAEDTAVYYCARDWWYPPYYW
GFDYWGQGTLVTVSS

CDR1: SYAMS
CDR2: AISGSGGSTYYADSVKG
CDR3: DWWYPPYYWGFDY

**MF3163:** heavy chain variable region sequence of an erbB-3 binding antibody Nucleic acid sequence (underlined sequence encodes end of leader peptide):

1 <u>GGCCCAGCCG GCCATGGCC</u>C AGGTGCAGCT GGTGCAGTCT GGGGCTGAGG TGAAGAAGCC

61 TGGGGCCTCA GTGAAGGTCT CCTGCAAGGC TTCTGGATAC ACCTTCACCG GCTACTATAT

121 GCACTGGGTG CGACAGGCCC CTGGACAAGG GCTTGAGTGG ATGGGATGGA TCAACCCTAA

181 CAGTGGTGGC ACAAACTATG CACAGAAGTT TCAGGGCAGG GTCACGATGA CCAGGGACAC

241 GTCCATCAGC ACAGCCTACA TGGAGCTGAG CAGGCTGAGA TCTGACGACA CGGCCGTGTA

301 TTACTGTGCA AAAGATTCTT ACTCTCGTCA TTTCTACTCT TGGTGGGCCT TTGATTATTG

361 GGGCCAAGGT ACCCTGGTCA CCGTCTCCAG T

Amino acid sequence:

QVQLVQSGAEVKKPGASVKVSCKASGYTFTGYYMHWVRQAPGQGLEWMGWI NPNSGGTNYAQKFQGRVTMTRDTSISTAYMELSRLRSDDTAVYYCAKDSYSRH FYSWWAFDYWGQGTLVTVSS

| | |
|---|---|
| CDR1: | GYYMH |
| CDR2: | WINPNSGGTNYAQKFQG |
| CDR3: | DSYSRHFYSWWAFDY |

**MF3099:** heavy chain variable region sequence of an erbB-3 binding antibody Nucleic acid sequence (underlined sequence encodes end of leader peptide):

1 <u>GGCCCAGCCG GCCATGGCC</u>G AGGTCCAGCT GCAGCAGCCT GGGGCTGAGC TGGTGAGGCC

61 TGGGACTTCA GTGAAGTTGT CCTGCAAGGC TTCTGGCTAC ACCTTCACCA GCTACTGGAT

121 GCACTGGGTA AAGCAGAGGC CTGGACAAGG CCTTGAGTGG ATCGGAATTC TTGATCCTTC

181 TGATAGTTAT ACTACCTACA ATCAAAAGTT CAAGGGCAAG GCCACATTAA CAGTAGACAC

241 ATCCTCCAGC ATAGCCTACA TGCAGCTCAG CAGCCTGACA TCTGAGGACT CTGCGCTCTA

301 TTACTGTGCA AGAGGGGGAG ATTACGACGA GGGAGGTGCT ATGGACTACT GGGGTCAAGG

361 AACCTCGGTC ACCGTCTCCA GT

Amino acid sequence:

EVQLQQPGAELVRPGTSVKLSCKASGYTFTSYWMHWVKQRPGQGLEWIGILDP
SDSYTTYNQKFKGKATLTVDTSSSIAYMQLSSLTSEDSALYYCARGGDYDEGGA
MDYWGQGTSVTVSS

CDR1: SYWMH
CDR2: ILDPSDSYTTYNQKFKG
CDR3: GGDYDEGGAMDY

**MF3307:** heavy chain variable region sequence of an erbB-3 binding antibody Nucleic acid sequence (underlined sequence encodes end of leader peptide):

```
1 GGCCCAGCCG GCCATGGCCC AGGTGCAGCT GGTGCAGTCT GGGGCTGAGG
TGAAGAAGCC
61 TGGGGCCTCA GTGAAGGTCT CCTGCAAGGC TTCTGGATAC ACCTTCACCG
GCTACTATAT
121 GCACTGGGTG CGACAGGCCC CTGGACAAGG GCTTGAGTGG ATGGGATGGA
TCAACCCTAA
181 CAGTGGTGGC ACAAACTATG CACAGAAGTT TCAGGGCAGG GTCACGATGA
CCAGGGACAC
241 GTCCATCAGC ACAGCCTACA TGGAGCTGAG CAGGCTGAGA TCTGACGACA
CGGCCGTGTA
301 TTACTGTGCA AGAGGTTCTC GTAAACGTCT GTCTAACTAC TTCAACGCCT
TTGATTATTG
361 GGGCCAAGGT ACCCTGGTCA CCGTCTCCAG T
```

Amino acid sequence:

QVQLVQSGAEVKKPGASVKVSCKASGYTFTGYYMHWVRQAPGQGLEWMGWI
NPNSGGTNYAQKFQGRVTMTRDTSISTAYMELSRLRSDDTAVYYCARGSRKRL
SNYFNAFDYWGQGTLVTVSS

CDR1: GYYMH
CDR2: WINPNSGGTNYAQKFQG
CDR3: GSRKRLSNYFNAFDY

**Sequences 1C**

Common Light Chain

The variable region of IGKV1-39

DIQMTQSPSSLSASVGDRVTITCRASQSISSYLNWYQQKPGKAPKLLIYAASSLQ
SGVPSRFSGSGSGTDFTLTISSLQPEDFATYYCQQSYSTP

CDR 1: RASQSISSYLN

(continued)

| | |
|---|---|
| CDR 2: | AASSLQS |
| CDR 3: | QQSYSTPPT |

IGKV1-39/jk1

DIQMTQSPSSLSASVGDRVTITCRASQSISSYLNWYQQKPGKAPKLLIYAASSLQ
SGVPSRFSGSGSGTDFTLTISSLQPEDFATYYCQQSYSTPPTFGQGTKVEIK

Common light chain IGKV1-39/jk1 (constant region is underligned)

DIQMTQSPSSLSASVGDRVTITCRASQSISSYLNWYQQKPGKAPKLLIYAASSLQ
SGVPSRFSGSGSGTDFTLTISSLQPEDFATYYCQQSYSTPPTFGQGTKVEIKRTV
AAPSVFIFPPSDEQLKSGTASVVCLLNNFYPREAKVQWKVDNALQSGNSQESVT
EQDSKDSTYSLSSTLTLSKADYEKHKVYACEVTHQGLSSPVTKSFNRGEC

IGKV1-39/jk5 common light chain variable domain

DIQMTQSPSSLSASVGDRVTITCRASQSISSYLNWYQQKPGKAPKLLIYAASSLQ
SGVPSRFSGSGSGTDFTLTISSLQPEDFATYYCQQSYSTPPITFGQGTRLEIK

**Sequences** 1D (erbB-2 specific)

heavy chain for erbB-2 binding

QVQLVQSGAEVKKPGASVKLSCKASGYTFTAYYINWVRQAPGQGLEWIGRIYPG
SGYTSYAQKFQGRATLTADESTSTAYMELSSLRSEDTAVYFCARPPVYYDSAWF
AYWGQGTLVTVSSASTKGPSVFPLAPSSKSTSGGTAALGCLVKDYFPEPVTVSW
NSGALTSGVHTFPAVLQSSGLYSLSSVVTVPSSSLGTQTYICNVNHKPSNTKVD
KRVEPKSCDKTHTCPPCPAPELLGGPSVFLFPPKPKDTLMISRTPEVTCVVVDV
SHEDPEVKFNWYVDGVEVHNAKTKPREEQYNSTYRVVSVLTVLHQDWLNGKE
YKCKVSNKALPAPIEKTISKAKGQPREPQVYTDPPSREEMTKNQVSLTCEVKGF
YPSDIAVEWESNGQPENNYKTTPPVLDSDGSFFLYSKLTVDKSRWQQGNVFSC
SVMHEALHNHYTQKSLSLSPG

heavy chain for erbB-3 binding

QVQLVQSGAEVKKPGASVKVSCKASGYTFTGYYMHWVRQAPGQGLEWMGWI
NPNSGGTNYAQKFQGRVTMTRDTSISTAYMELSRLRSDDTAVYYCARDHGSRH
FWSYWGFDYWGQGTLVTVSSASTKGPSVFPLAPSSKSTSGGTAALGCLVKDYF
PEPVTVSWNSGALTSGVHTFPAVLQSSGLYSLSSVVTVPSSSLGTQTYICNVNHK
PSNTKVDKRVEPKSCDKTHTCPPCPAPELLGGPSVFLFPPKPKDTLMISRTPEV
TCVVVDVSHEDPEVKFNWYVDGVEVHNAKTKPREEQYNSTYRVVSVLTVLHQ
DWLNGKEYKCKVSNKALPAPIEKTISKAKGQPREPQVYTKPPSREEMTKNQVS
LKCLVKGFYPSDIAVEWESNGQPENNYKTTPPVLDSDGSFFLYSKLTVDKSRW
QQGNVFSCSVMHEALHNHYTQKSLSLSPG

**Sequences 1E**

**HER2-specific Ab sequences**

**MF2889:** heavy chain variable region sequence of an erbB-2 binding antibody Nucleic acid sequence (underlined sequence encodes end of leader peptide):

```
1   GGCCCAGCCG GCCATGGCCG AGGTCCAGCT GCAGCAGTCT GGAGCTGAGC TGGTAAGGCC
61  TGGGACTTCA GTGAAGGTGT CCTGCAAGGC TTCTGGATAC GCCTTCACTA ATTATTTGAT
121 AGAGTGGGTA AAGCAGAGGC CTGGCCAGGG CCTTGAGTGG ATTGGAGTGA TTTATCCTGA
181 AGGTGGTGGT ACTATCTACA ATGAGAAGTT CAAGGGCAAG GCAACACTGA CTGCAGACAA
241 ATCCTCCAGC ACTGCCTACA TGCAGCTCAG CGGCCTGACA TCTGAGGACT CTGCGGTCTA
301 TTTCTGTGCA AGAGGAGACT ATGATTACAA ATATGCTATG GACTACTGGG GTCAAGGAAC
361 CTCGGTCACC GTCTCCAGT
```

Amino acid sequence:

EVQLQQSGAELVRPGTSVKVSCKASGYAFTNYLIEWVKQRPGQGLEWIGVIYPE
GGGTIYNEKFKGKATLTADKSSSTAYMQLSGLTSEDSAVYFCARGDYDYKYAM
DYWGQGTSVTVSS

| | |
|---|---|
| CDR1: | NYLIE |
| CDR2: | VIYPEGGGTIYNEKFKG |
| CDR3: | GDYDYKYAMDY |

**MF2913:** heavy chain variable region sequence of an erbB-2 binding antibody Nucleic acid sequence (underlined sequence encodes end of leader peptide):

```
  1 GGCCCAGCCG GCCATGGCCG AGGTCAAGCT GCAGCAGTCT GGACCTGAGC TGGTGAAGCC
 61 TGGCGCTTCA GTGAAGATAT CCTGCAAGGC TTCTGGTTAC TCATTCACTG ACTACAAAAT
121 GGACTGGGTG AAGCAGAGCC ATGGAAAGAG CCTCGAATGG ATTGGAAATA TTAATCCTAA
181 CAGTGGTGGT GTTATCTACA ACCAGAAGTT CAGGGGCAAG GTCACATTGA CTGTTGACAG
241 GTCCTCCAGC GCAGCCTACA TGGAGCTCCG CAGCCTGACA TCTGAGGACA CTGCAGTCTA
301 TTATTGTTCA AGAGGACTGT GGGATGCTAT GGACTCCTGG GGTCAAGGAA CCTCGGTCAC
361 CGTCTCCAGT
```

Amino acid sequence:

EVKLQQSGPELVKPGASVKISCKASGYSFTDYKMDWVKQSHGKSLEWIGNINP
NSGGVIYNQKFRGKVTLTVDRSSSAAYMELRSLTSEDTAVYYCSRGLWDAMDS
WGQGTSVTVSS

| | |
|---|---|
| CDR1: | DYKMDWVKQSHGKSLE |
| CDR2: | NQKFRG |
| CDR3: | GLWDAMDS |

**MF1847:** heavy chain variable region sequence of an erbB-2 binding antibody Nucleic acid sequence (underlined sequence encodes end of leader peptide):

```
1   GGCCCAGCCG GCCATGGCCC AGGTGCAGCT GGTGGAGTCT GGGGGAGGCG TGGTCCAGCC
61  TGGGAGGTCC CTGAGACTCT CCTGTGCAGC CTCTGGATTC ACCTTCAGTA GCTATGGCAT
121 GCACTGGGTC CGCCAGGCTC CAGGCAAGGG GCTGGAGTGG GTGGCAGTTA TATCATATGA
181 TGGAAGTAAT AAATACTATG CAGACTCCGT GAAGGGCCGA TTCACCATCT CCAGAGACAA
241 TTCCAAGAAC ACGCTGTATC TGCAAATGAA CAGCCTGAGA GCTGAGGACA CGGCCGTGTA
301 TTACTGTGCA AAAGGTTGGT GGCATCCGCT GCTGTCTGGC TTTGATTATT GGGGCCAAGG
361 TACCCTGGTC ACCGTCTCCA GT
```

Amino acid sequence:

QVQLVESGGGVVQPGRSLRLSCAASGFTFSSYGMHWVRQAPGKGLEWVAVISY
DGSNKYYADSVKGRFTISRDNSKNTLYLQMNSLRAEDTAVYYCAKGWWHPLLS
GFDYWGQGTLVTVSS

| | |
|---|---|
| CDR1: | SYGMH |
| CDR2: | VISYDGSNKYYADSVKG |
| CDR3: | GWWHPLLSGFDY |

**MF3001:** heavy chain variable region sequence of an erbB-2 binding antibody Nucleic acid sequence (underlined sequence encodes end of leader peptide):

```
1   GGCCCAGCCG GCCATGGCCG AGGTCCAGCT GCAGCAGTCT GGGGCTGAAC TGGCAAAACC
61  TGGGGCCTCA GTGAAGCTGT CCTGCAAGAC TTCTGGCTAC AACTTTCCTA TCTACTGGAT
121 GCACTGGGTA AAACAGAGGC CTGGACGGGG TCTGGAATGG ATTGGATACA TTAATCCTAG
181 TACTGGTTAT ATTAAGAACA ATCAGAAGTT CAAGGACAAG GCCACCTTGA CTGCAGACAA
241 ATCCTCCAAC ACAGCCTACA TGCAGCTGAA CAGCCTGACA TATGAGGACT CTGCAGTCTA
301 TTACTGTACA AGAGAAGGGA TAACTGGGTT TACTTACTGG GGCCAAGGGA CTCTGGTCAC
361 CGTCTCCAGT
```

Amino acid sequence:

EVQLQQSGAELAKPGASVKLSCKTSGYNFPIYWMHWVKQRPGRGLEWIGYINP STGYIKNNQKFKDKATLTADKSSNTAYMQLNSLTYEDSAVYYCTREGITGFTY WGQGTLVTVSS

| | |
|---|---|
| CDR1: | IYWMHWVKQRPGRGLE |
| CDR2: | NQKFKD |
| CDR3: | EGITGFTY |

**MF1898:** heavy chain variable region sequence of an erbB-2 binding antibody Nucleic acid sequence (underlined sequence encodes end of leader peptide):

```
1   GGCCCAGCCG GCCATGGCCC AGGTGCAGCT GGTGGAGTCT GGGGGAGGCG TGGTCCAGCC
61  TGGGAGGTCC CTGAGACTCT CCTGTGCAGC CTCTGGATTC ACCTTCAGTA GCTATGGCAT
121 GCACTGGGTC CGCCAGGCTC CAGGCAAGGG GCTGGAGTGG GTGGCAGTTA TATCATATGA
181 TGGAAGTAAT AAATACTATG CAGACTCCGT GAAGGGCCGA TTCACCATCT CCAGAGACAA
241 TTCCAAGAAC ACGCTGTATC TGCAAATGAA CAGCCTGAGA GCTGAGGACA CGGCCGTGTA
301 TTACTGTGCA AAAGATGGTT TCCGTCGTAC TACTCTGTCT GGCTTTGATT ATTGGGGCCA
361 AGGTACCCTG GTCACCGTCT CCAGT
```

Amino acid sequence:

QVQLVESGGGVVQPGRSLRLSCAASGFTFSSYGMHWVRQAPGKGLEWVAVISY DGSNKYYADSVKGRFTISRDNSKNTLYLQMNSLRAEDTAVYYCAKDGFRRTTL SGFDYWGQGTLVTVSS

| | |
|---|---|
| CDR1: | SYGMH |
| CDR2: | VISYDGSNKYYADSVKG |
| CDR3: | DGFRRTTLSGFDY |

**MF3003** heavy chain variable region sequence of an erbB-2 binding antibody Nucleic acid sequence (underlined sequence encodes end of leader peptide):

```
1   GGCCCAGCCG GCCATGGCCC AGGTGCAGCT GAAGCAGTCT GGACCTGAGC TGGTGAAGCC
61  TGGGGCCTCA GTGAAGATTT CCTGCAAGGC TTCTGGCGAC GCATTCAGTT ACTCCTGGAT
121 GAACTGGGTG AAGCAGAGGC CTGGAAAGGG TCTTGAGTGG ATTGGACGGA TTTATCCTGG
181 AGATGGAGAT ATTAACTACA ATGGGAAGTT CAAGGGCAAG GCCACACTGA CTGCAGACAA
241 ATCCTCCAGC ACAGCCCACC TGCAACTCAA CAGCCTGACA TCTGAGGACT CTGCGGTCTA
301 CTTCTGTGCA AGAGGACAGC TCGGACTAGA GGCCTGGTTT GCTTATTGGG GCCAGGGGAC
361 TCTGGTCACC GTCTCCAGT
```

Amino acid sequence:

QVQLKQSGPELVKPGASVKISCKASGDAFSYSWMNWVKQRPGKGLEWIGRIYPGDGDINYNGKFKGKATLTADKSSSTAHLQLNSLTSEDSAVYFCARGQLGLEAWFAYWGQGTLVTVSS

| | |
|---|---|
| CDR1: | YSWMNWVKQRPGKGLE |
| CDR2: | NGKFKG |
| CDR3: | GQLGLEAWFAY |

**HER3-specific Ab sequences**

**MF6058:** heavy chain variable region sequence of an erbB-3 binding antibody Nucleic acid sequence (underlined sequence encodes end of leader peptide):

```
1   GGCCCAGCCG GCCATGGCCC AGGTGCAGCT GGTGCAGTCT GGGGCTGACG TGAAGAAGCC
61  TGGGGCCTCA GTGAAGGTCA CGTGCAAGGC TTCTGGATAC ACCTTCACCG GCTACTATAT
121 GCACTGGGTG CGACAGGCCC CTGGACAAGC TCTTGAGTGG ATGGGATGGA TCAACCCTCA
181 AAGTGGTGGC ACAAACTATG CAAAGAAGTT TCAGGGCAGG GTCTCTATGA CCAGGGAGAC
241 GTCCACAAGC ACAGCCTACA TGCAGCTGAG CAGGCTGAGA TCTGACGACA CGGCTACGTA
301 TTACTGTGCA AGAGATCATG GTTCTCGTCA TTTCTGGTCT TACTGGGGCT TTGATTATTG
361 GGGCCAAGGT ACCCTGGTCA CCGTCTCCAG T
```

Amino acid sequence:

QVQLVQSGADVKKPGASVKVTCKASGYTFTGYYMHWVRQAPGQALEWMGWINPQSGGTNYAKKFQGRVSMTRETSTSTAYMQLSRLRSDDTATYYCARDHGSRHFWSYWGFDYWGQGTLVTVSS

| | |
|---|---|
| CDR1: | GYYMH |
| CDR2: | WINPQSGGTNYAKKFQG |

(continued)

CDR3: DHGSRHFWSYWGFDY

**MF6061:** heavy chain variable region sequence of an erbB-3 binding antibody Nucleic acid sequence (underlined sequence encodes end of leader peptide):

```
1 GGCCCAGCCG GCCATGGCCC AGGTGCAGCT GGTGCAGTCT GGGGCTGAGG
TGAAGAAGCC
61 TGGGGCCTCA GTGAAGGTCT CCTGCAAGGC TTCTGGATAC ACCTTCACCG
GCTACTATAT
121 GCACTGGGTG CGACAGGCCC CTGGACAAGG GCTTGAGTGG ATGGGATGGA
TCAACCCTCA
181 GAGTGGTGGC ACAAACTATG CACAGAAGTT TAAGGGCAGG GTCACGATGA
CCAGGGACAC
241 GTCCACCAGC ACAGCCTACA TGGAGCTGAG CAGGCTGAGA TCTGACGACA
CGGCTGTGTA
301 TTACTGTGCA AGAGATCATG GTTCTCGTCA TTTCTGGTCT TACTGGGGCT
TTGATTATTG
361 GGGCCAAGGT ACCCTGGTCA CCGTCTCCAG T
```

Amino acid sequence:

QVQLVQSGAEVKKPGASVKVSCKASGYTFTGYYMHWVRQAPGQGLEWMGWI
NPQSGGTNYAQKFKGRVTMTRDTSTSTAYMELSRLRSDDTAVYYCARDHGSRH
FWSYWGFDYWGQGTLVTVSS

CDR1: GYYMH
CDR2: WINPQSGGTNYAQKFKG
CDR3: DHGSRHFWSYWGFDY

**MF6065:** heavy chain variable region sequence of an erbB-3 binding antibody Nucleic acid sequence (underlined sequence encodes end of leader peptide):

```
     1 GGCCCAGCCG GCCATGGCCC AGGTGCAGCT GGTGCAGTCT GGGGCTGAGG
TGAAGAAGCC
    61 TGGGGCCTCA GTGAAGGTCT CCTGCAAGGC TTCTGGATAC ACCTTCACCT
CTTACTATAT
   121 GCACTGGGTG CGACAGGCCC CTGGACAAGG GCTTGAGTGG ATGGGATGGA
TCAACCCTCA
   181 GGGGGGTTCT ACAAACTATG CACAGAAGTT TCAGGGCAGG GTCACGATGA
CCAGGGACAC
   241 GTCCACCAGC ACAGTGTACA TGGAGCTGAG CAGGCTGAGA TCTGAGGACA
CGGCTGTGTA
   301 TTACTGTGCA AGAGATCATG GTTCTCGTCA TTTCTGGTCT TACTGGGGCT
TTGATTATTG
   361 GGGCCAAGGT ACCCTGGTCA CCGTCTCCAG T
```

Amino acid sequence:

QVQLVQSGAEVKKPGASVKVSCKASGYTFTSYYMHWVRQAPGQGLEWMGWIN
PQGGSTNYAQKFQGRVTMTRDTSTSTVYMELSRLRSEDTAVYYCARDHGSRHF
WSYWGFDYWGQGTLVTVSS

| | |
|---|---|
| CDR1: | SYYMH |
| CDR2: | WINPQGGSTNYAQKFQG |
| CDR3: | DHGSRHFWSYWGFDY |

For the purpose of clarity and a concise description features are described herein as part of the same or separate embodiments, however, it will be appreciated that the scope of the invention may include embodiments having combinations of all or some of the features described.

**BRIEF DESCRIPTION OF THE DRAWINGS**

Figure 1 : Amino acid alignment of MF3178 variants. Dots indicate the same amino acid as in MF3178 at that position. The CDR1, CDR2 and CDR3 sequences of MF3178 are in bold and underlined.

Figure 2: Increased *in* vivo tumor-targeting of bispecific over monoclonal antibody. Micro-PET imaging demonstrates that the PB4188 variant more effectively accumulates in tumors compared to the HER3 monoclonal (Fig. 2A). Gamma-counter quantification of radioactivity present in tumors confirmed that levels of PB4188 variant in the tumors were 2.5-fold higher than for the parental anti-HER3 antibody (Fig. 2B). Quantitative biodistribution for tumour uptake in the 4 mAb groups at 48 hrs. Results are expressed as a percentage of the injected dose per gram of tissue (%ID/g), error bars indicate ±S (Fig. 2C)

Figure 3: Cartoon illustration of the three situations taken into account in the model. HER2 is colored orange and HER3 is yellow.

Figure 4: Normalized PL: average amount of HRG bound to HER3 normalized for the total number of available binding sites; HER2/HER3 copy number ratio is 1:1 and HRG concentration is 1nM (Fig 4A) or 10nM (Fig 4B) (Fig(1 or 100). HER2/HER3 copy number ratio is 100:1 and HRG concentration is 1nM (Fig 4C) or 10nM (Fig 4D).

Figure 5: States modelled for monospecific anti-HER3 (A) and bispecific anti-HER3/HER2 (B).

Figure 6: Antibody antagonist mode dose response curves in EGFR:HER2, HER2:HER3 and HER2:HER4 assays. Reporter cells were seeded for 4hr at 37°C at 2.5K/well in the case of EGFR:HER2 or 5K/well in the case of HER2:HER3 and HER2:HER4. Antibodies were serially diluted and incubated for 3hr at 37°C prior to stimulation for 16 hrs with 10ng/ml EGF or 30 ng/ml HRG-β2 in the case of EGFR:HER2 or HER2:HER3 and HER2:HER4, respectively. Reference stimulation curves of agonists were obtained by incubating titrations of ligands alone for 24 hrs. Each data point represents the mean and standard deviation of four replicates per dose. Data were plotted in GraphPad Prism and curve fits were performed using a log (inhibitor) vs response- variable response (4 parameters) fit to calculate IC50's.

## EXAMPLES

### Example 1: ErbB-2-guided targeting

An imaging experiment was performed comparing the HER2xHER3 bispecific antibody (PB4188) to the HER3 bivalent monoclonal antibody. Variants of bAb PB4188 and anti-HER3 MF3178 (parental antibody) were labelled with 64Cu and injected intravenously in mouse xenografted with HER2 gene-amplified JIMT-1 tumors. Micro-PET imaging demonstrated that the PB4188 variant more effectively accumulated in tumors compared to the HER3 monoclonal (Fig. 2A). Gamma-counter quantification of radioactivity present in tumors confirmed that levels of PB4188 variant in the tumors were 2.5-fold higher than for the parental anti-HER3 antibody (Fig. 2B). Overall, in vitro and in vivo data demonstrate that HER2-targeting is responsible for enhanced binding of PB4188 on tumor cells. Additional studies were performed using an anti-HER2 (MF3958)) antibody. Figure 2C summarizes the results of the respective antibodies labelledwith 64Cu and injected in mouse xenografted with HER2 gene-amplified JIMT-1 tumors (n=4 mice for each antibody treatment).

Methods

Biodistribution study. Variants of bAb PB4188, anti-HER2 MF3958, and anti-HER3 MF3178 were conjugated to a bifunctional chelator [Paterson 2014 Dalton Transactions]. Binding characteristics of the conjugated products to the target were confirmed using flow cytometry-based assays. Proteins were then labelled with 64Cu and mice bearing JIMT-1 breast xenografts were administered the radiolabeled antibodies via tail vein (Figure 2A-B and "i.v." for Figure 2C) or intraperitoneal ("i.p." for Figure 2C). MicroPET/CT images were acquired 48 hrs post-injection, after which tumor were excised and radioactivity was measured in a gamma counter. Results were expressed as percentage injected dose per gram tissue.

### Example 2: Model binding

We used the grand canonical formalism to model the effect of the bispecific antibody MCLA128 (afucosylated version of PB4188; MF3958 as ErbB-2 arm and MF3178 as ErbB-3 arm) and compared it with a monospecific anti-HER3 with two Fab MF3178 arms. Under the approximation of the grand canonical ensemble the chemical potential ($\mu$), the volume (V) and the temperature (T) are kept constant while the number of molecules can vary. This approach has been widely used in surface science [Clark et al. 2006].

The basis of the method is to simulate a series of states where the ligand and the antibody are in solution and their concentration can vary, while the receptors are fixed on a surface. The receptors, the ligand and the antibody are considered as rigid models [Weinert et al. 2014] and binding of the antibody and the ligand to the antigens sites is uncorrelated. Moreover, in this model random mixing is assumed (each state is equally probable).

Although in real life you have conformational changes leading to signal activation in a dynamic system like cellular membrane, the grand canonical ensemble provides a simplified approach that accounts for essential variables such as number of receptors on the surface, ligand/antibody concentrations (HRG and A)and binding affinities.

The possible situations in which the receptors can be found on the surface are approximated as: 2 copies of HER2, 2 copies of HER3 or 1 copy of HER2 and 1 copy of HER3 (Figure 3). The interactions taken into account are: the binding of HRG to HER3 with $K_D$= 0.2 nM, the binding of a monospecific anti-HER3 antibody with two FabMF3178 arms with Kd=0.2 nM for each arm and binding of a bispecific antibody with FabMF3958 binding to HER2 with Kd=2 nM and FabMF3178 binding to HER3 with Kd=0.2 nM. The Kds correspond to the energy contribution of each interaction.

The states taken into account to generate the grand canonical partition function represent all the ways in which the antibody (either monospecific or bispecific) and the ligand (HRG) can bind to the three paired situations listed in Figure 4.

The output of the model is the average amount of HRG bound to HER3 at determinate conditions of antibody (bispecific or monospecific), concentration of antibody (1-100 nM), HRG concentration (1 or 10 nM) and HER2/HER3 copy number ratio (1 or 100).

In Figure 4, the average amount of HRG bound to HER3 is plotted against increasing amounts of antibody. We modelled this relation for two different concentrations of HRG: low HRG (1 nM) and stress conditions (10 nM) and for two different cell lines, varying copy number of HER2 (i.e. in ratio of HER2:HER3 equals 1 or 100).

The plots in Figure 4 show that in cell types with a copy number of HER2 equal to HER3 there is little advantage of the bispecific antibody respect to the monospecific anti-HER3 and the monospecific performs (slightly) better in competing with HRG both a low and high HRG concentrations (Figure 4A-C). On the other hand when there is an excess of HER2 (100xHER3), the bispecific is much better than the monospecific in competing for the HRG binding both at low and high HRG concentrations (Figure 4B-D). The outcome of the model proves in a simple but elegant way how the higher number of HER2 copies gives a tremendous advantage to the bispecific antibody in respect to a monoclonal anti-HER3. In fact, the bispecific Ab will always be able to bind HER2 no matter how much HRG is present, while the monoclonal HER3 will suffer from competition from HRG binding especially at stress conditions.

Methods

Figure 5 depicts the states modelled and their respective weights

Main equations

$$P_l = \frac{< N_l >}{N_{tot}} = \lambda_l \left(\frac{d \ln \Xi}{d\lambda_l}\right)_{\lambda_a}$$

$$\Xi = p_{22}\Xi_{22} + p_{33}\Xi_{33} + p_{23}\Xi_{23}$$

$$e^{-2\beta\varepsilon_i} = (e^{-\beta\varepsilon_i})^2 = K_{A_i}^2 = \frac{1}{K_{D_i}^2}$$

$$p_{22} = \frac{N_2^2}{N_{tot}^2}$$

$$p_{33} = \frac{N_3^2}{N_{tot}^2}$$

$$p_{23} = 2\frac{N_2 N_3}{N_{tot}^2}$$

$\Xi$ = grand partition function
$\varepsilon$ = binding energy
$\lambda_i$ = fugacity ~ $c_i$
$p_{ii}$ = probability associated to that state

$$N_{tot} = N_2 + N_3$$

$$N_3 = 30{,}000$$

$$N_2 = 30{,}000 / 3{,}000{,}000$$

State equations for monoclonal anti-HER3 model

$$\Xi_{22} = 1$$

$$\Xi_{33} = 1 + 2\frac{\lambda_a}{K_{D_3}} + \frac{\lambda_a}{K_{D_3}^2} + 2\frac{\lambda_a\lambda_l}{K_{D_3}K_{D_l}} + \frac{\lambda_l^2}{K_{D_l}^2} + 2\frac{\lambda_a^2}{K_{D_3}^2}$$

$$\Xi_{23} = 1 + \frac{\lambda_a}{K_{D_3}} + \frac{\lambda_l}{K_{D_l}}$$

State equations for bispecific anti-HER2/HER3 model

$$\Xi_{22} = 1 + 2\frac{\lambda_a}{K_{D_2}} + \frac{\lambda_a^2}{K_{D_2}^2}$$

$$\Xi_{33} = 1 + 2\frac{\lambda_a}{K_{D_3}} + 2\frac{\lambda_a\lambda_l}{K_{D_3}K_{D_l}} + \frac{\lambda_l^2}{K_{D_l}^2} + 2\frac{\lambda_l}{K_{D_l}} + \frac{\lambda_a^2}{K_{D_3}^2}$$

$$\Xi_{23} = 1 + \frac{\lambda_a}{K_{D_3}} + \frac{\lambda_a}{K_{D_2}} + \frac{\lambda_a}{K_{D_2}K_{D_3}} + \frac{\lambda_l}{K_{D_l}} + \frac{\lambda_a\lambda_l}{K_{D_2}K_{D_l}} + \frac{\lambda_a^2}{K_{D_2}K_{D_3}}$$

**Example 3 Inhibition of heterodimer formation**

Heterodimerization assays based on the enzyme fragment complementation technology were used. The β-galactosidase enzyme can be artificially split into two inactive fragments, the enzyme donor and the enzyme acceptor, which combine into an active enzyme only when in close proximity. Each sequence encoding either the enzyme donor or the enzyme acceptor is linked to the extracellular and transmembrane domains of each heterodimerization partner. Both genes are then co-transfected in U2OS cells to express extracellular domains of RTK receptors linked to one domain of β-galactosidase (ED or EA). Upon agonistic stimulation of one RTK receptor, both RTK receptors dimerize, inducing formation of an active fully reconstituted β -galactosidase enzyme. Ultimately, β -galactosidase activity is measured by adding a substrate that upon hydrolyzation will lead to light emission.

Antibodies where tested in EGFR:HER2, HER2:HER3 and HER3:HER4 heterodimerization reporter cell lines. RTK heterodimerization assays were run with the bispecific antibody MCLA-128 (MF3178 arm and MF3958 arm); anti-HER3 antibodies MF3178/PG3178 and PG3793/AMG-888/ patritumab; and anti-HER2 antibodies MF3958/PG3958, PG2867/trastuzumab, PG2869/pertuzumab, and Perjeta (clinical batch of pertuzumab). EGF and HRG titrations in EGFR:HER2 and HER2:HER3, HER2:HER4 assays showed dose-dependent agonist responses (Figure 5). MCLA-128 showed complete inhibition of HER2:HER3 dimer formation specifically and had no effect on EGFR:HER2 or HER2:HER4 heterodimerization. In contrast, trastuzumab (PG2867) behaved as partial antagonist in EGFR:HER2 and HER2:HER3 assays.

MCLA-128 and PG3178 fully inhibited HRG-induced HER2:HER3 dimerization with the highest potency (Table 1).

Table 1: Summary results of EC50 of antibodies tested in RTK heterodimerization assays. EC50 were determined non-linear regressions (4 parameters) in Prism.

| IC50 (nM) | EGFR:HER2 | HER2:HER3 | HER2:HER4 |
|---|---|---|---|
| **PG1337** | - | - | - |
| **PB4188** | - | 1.12 | - |
| **PG3187** | - | 1.27 | - |
| **PG3958** | - | - | 1.69 |
| **PG2867** | 0.30 | 4.91 | 0.63 |

(continued)

| IC50 (nM) | EGFR:HER2 | HER2:HER3 | HER2:HER4 |
|---|---|---|---|
| **PG2869** | 0.47 | 4.22 | 2.91 |
| **PG3793** | - | 3.23 | - |
| **Perjeta** | 0.61 | 6.26 | 5.44 |
| **Agonist** | 0.02 | 0.22 | 0.08 |

The potency of trastuzumab was about 4-fold lower than MCLA-128 or PG3178 in the HER2:HER3 assay. Perjeta (clinical pertuzumab) behaved as full antagonist in all three assays and gave a similar profile as PG2867 (pertuzumab). In HER2:HER4 assays, both anti-HER2 PG3958 and PG2867 (pertuzumab) showed minor decreases in dimerization that appeared to be dose-dependent. Small nonspecific responses in EGFR:HER2 assays were observed at high concentrations of PG1337, MCLA-128, PG3178 and PG3958.

MCLA-128 showed specific inhibition of HER2:HER3 heterodimers only. This indicates that upon binding on HER2, MCLA-128 should not sterically impair interaction of HER2 with EGFR upon EGF stimulation, nor impair heterodimerization of HER2 with HER4 upon HRG stimulation.

The latter is in line with observation in the HRG-induced cell cycle-based proliferation assay of T47D cells. Assays using these cells failed to demonstrate inhibitory activity of MCLA-128 or PG3178, which was presumably attributed to the higher expression of HER4 compared to HER3. HRG is thought to preferably signal via HER2:HER4 in T47D cells instead of HER2:HER3, explaining the lack of efficacy of MCLA-128 and indicating a specificity of MCLA-128 for HRG-induced HER2:HER3 dimers and not for HRG-induced HER2:HER4 dimers.

In the current study, trastuzumab blocked EGF- and HRG-induced heterodimerization of EGFR:HER2 and HER2:HER3, respectively. Trastuzumab and pertuzumab behaved as partial and full antagonist, respectively, which is in line with the generally accepted claim that trastuzumab blocks ligand-independent activation of HER2 while pertuzumab inhibits ligand-dependent signaling. The fact that a trastuzumab inhibitory response is observed in these assays might be due to the overexpression of both targets. This might allow a more sensitive readout than traditional immunoprecipitation experiments.

Finally, while PG3793 showed a lower binding affinity than PG3178 on MCF-7, its lower potency in HER2:HER3 heterodimerization assay is less severe (2.5-fold difference in dimerization assay potency versus 30-fold difference in binding assay affinity). This discrepancy between binding affinity and antagonism potency has previously been observed in the case of MCLA-128 and PG3178. While PG3178 binds MCF-7 with a slightly better affinity than MCLA-128, MCLA-128 outperforms PG3178 in a cell cycle-based proliferation assay.

## References

Yarden Y, Pines G.2012. The ERBB network: at last, cancer therapy meets systems biology. Nat Rev CancerJul 12;12(8):553-63.

Wilson TR, Fridlyand J, Yan Y, Penuel E, Burton L, Chan E, Peng J, Lin E, Wang Y, Sosman J, Ribas A, Li J, Moffat J, Sutherlin DP, Koeppen H, Merchant M, Neve R, Settleman J. 2012. Widespread potential for growth-factor-driven resistance to anticancer kinase inhibitors. Nature. Jul 26;487(7408):505-9.

Balko JM, Miller TW, Morrison MM, Hutchinson K, Young C, Rinehart C, Sánchez V, Jee D, Polyak K, Prat A, Perou CM, Arteaga CL, Cook RS. 2012. The receptor tyrosine kinase ErbB3 maintains the balance between luminal and basal breast epithelium. Proc Natl Acad Sci U S A. Jan 3;109(1):221-6.

Zhang H, Berezov A, Wang Q, Zhang G, Drebin J, Murali R, Greene MI. 2007. ErbB receptors: from oncogenes to targeted cancer therapies. J Clin Invest. Aug;117(8):2051-8.

Sergina NV, Rausch M, Wang D, Blair J, Hann B, Shokat KM, Moasser MM. 2007. Escape from HER-family tyrosine kinase inhibitor therapy by the kinase-inactive HER3. Nature. Jan 25;445(7126):437-41.

Junttila TT, Akita RW, Parsons K, Fields C, Lewis Phillips GD, Friedman LS, Sampath D, Sliwkowski MX. 2009. Ligand-independent HER2/HER3/PI3K complex is disrupted by trastuzumab and is effectively inhibited by the PI3K inhibitor GDC-0941. Cancer Cell. May 5;15(5):429-40.

Ocana A, Vera-Badillo F, Seruga B, Templeton A, Pandiella A, Amir E. 2013. HER3 overexpression and survival in solid tumors: a meta-analysis. J Natl Cancer Inst. Feb 20;105(4):266-73.

Junttila, T. T., K. Parsons, et al. (2010). "Superior In vivo Efficacy of Afucosylated Trastuzumab in the Treatment of HER2-Amplified Breast Cancer." Cancer Research 70(11): 4481-4489

Merchant et al. Nature Biotechnology, Vol. 16 July 1998 pp 677-681

## Claims

1. A bispecific antibody comprising a first antigen-binding site that binds ErbB-2 and a second antigen-binding site that binds ErbB-3, wherein the antibody can reduce a ligand-induced receptor function of ErbB-3 on a ErbB-2 and ErbB-3 positive cell, for use in the treatment of a subject having an ErbB-2/ErbB-3 positive tumor,
wherein said ErbB-2/ErbB-3 positive tumor has at least 150,000 ErbB-2 cell-surface receptors per cell and wherein said subject has not previously been treated with a ErbB-2 specific therapy or with a ErbB-3 specific therapy.

2. The bispecific antibody for use of claim 1, wherein the ErbB-2 therapy is an ErbB-2 specific antibody, preferably wherein the ErbB-2 specific antibody is trastuzumab or pertuzumab.

3. The bispecific antibody for use of claim 1 or 2, wherein the ErbB-3 therapy is a ErbB-3 specific antibody.

4. A bispecific antibody comprising a first antigen-binding site that binds ErbB-2 and a second antigen-binding site that binds ErbB-3, wherein the antibody can reduce a ligand-induced receptor function of ErbB-3 on a ErbB-2 and ErbB-3 positive cell, for use in the treatment of a subject having an ErbB-2/ErbB-3 positive tumor,
wherein said ErbB-2/ErbB-3 positive tumor has a ratio of ErbB-2/ErbB-3 cell-surface receptors per cell of at least 10:1.

5. The bispecific antibody for use according to claim 4 wherein said ErbB-2/ErbB-3 positive tumor has a ratio of ErbB-2/ErbB-3 cell-surface receptors per cell of at least 100:1.

6. A bispecific antibody comprising a first antigen-binding site that binds ErbB-2 and a second antigen-binding site that binds ErbB-3, wherein the antibody can reduce a ligand-induced receptor function of ErbB-3 on a ErbB-2 and ErbB-3 positive cell, for use in the treatment of a subject having an ErbB-2/ErbB-3 positive tumor,
wherein said ErbB-2/ErbB-3 positive tumor has a ratio of ErbB-1/ErbB-2 cell-surface receptors per cell of no more than 4:10, preferably no more than 2:10.

7. The bispecific antibody for use according to any of the preceding claims, wherein said ErbB-2/ErbB-3 positive tumor has at least 150,000 ErbB-2 cell-surface receptors per cell, preferably wherein said ErbB-2/ErbB-3 positive tumor has at least 1,000,000 ErbB-2 cell-surface receptors per cell.

8. The bispecific antibody for use according to any one of the preceding claims, wherein said ErbB-2/ErbB-3 positive tumor has less than 50,000 ErbB-3 cell-surface receptors per cell.

9. The bispecific antibody for use according to any one of the preceding claims, wherein said ErbB-2/ErbB-3 positive tumor has less than 400,000 ErbB-1 cell-surface receptors per cell, preferably less than 200,000 ErbB-1 cell-surface receptors per cell.

10. The bispecific antibody for use according to any one of the preceding claims, wherein the the ErbB-1 cell-surface receptor density, ErbB-2 cell-surface receptor density, and/or ErbB-3 cell-surface receptor density for said tumor is determined.

11. The bispecific antibody for use according to any one of the preceding claims, wherein said treatment further comprises the use of ErbB-1 inhibitor for treating said tumor.

12. The bispecific antibody for use according to any one of the preceding claims, wherein said first antigen-binding site binds domain I of ErbB-2 and said second antigen-binding site binds domain III of ErbB-3, preferably wherein the affinity of the first antigen-binding site for ErbB-2 is lower than the affinity of the second antigen-binding site for ErbB-3.

13. The bispecific antibody for use according to claim 12, wherein said antibody comprises

    i) at least the CDR1, CDR2 and CDR3 sequences of an ErbB-2 specific heavy chain variable region selected from the group consisting of MF2926, MF2930, MF1849; MF2973, MF3004, MF3958, MF2971, MF3025, MF2916, MF3991, MF3031, MF2889, MF2913, MF1847, MF3001, MF3003 and MF1898 or wherein said antibody comprises CDR sequences that differ in at most 3 amino acids, preferably in at most 2 amino acids, preferably in at most 1 amino acid from the CDR1, CDR2 and CDR3 sequences of MF2926, MF2930, MF1849; MF2973, MF3004, MF3958, MF2971, MF3025, MF2916, MF3991, MF3031, MF2889, MF2913, MF1847, MF3001, MF3003 or MF1898; and/or

ii) at least the CDR1, CDR2 and CDR3 sequences of an ErbB-3 specific heavy chain variable region selected from the group consisting of MF3178; MF3176; MF3163; MF3099; MF3307; MF6055; MF6056; MF6057; MF6058; MF6059; MF6060; MF6061; MF6062; MF6063; MF6064; MF 6065; MF6066; MF6067; MF6068; MF6069; MF6070; MF6071; MF6072; MF6073 and MF6074, or wherein said antibody comprises CDR sequences that differ in at most 3 amino acids, preferably in at most 2 amino acids, preferably in at most 1 amino acid from the CDR1, CDR2 and CDR3 sequences of MF3178; MF3176; MF3163; MF3099; MF3307; MF6055; MF6056; MF6057; MF6058; MF6059; MF6060; MF6061; MF6062; MF6063; MF6064; MF 6065; MF6066; MF6067; MF6068; MF6069; MF6070; MF6071; MF6072; MF6073 or MF6074; preferably wherein said antibody comprises

i) an ErbB-2 specific heavy chain variable region sequence selected from the group consisting of the heavy chain variable region sequences of MF2926, MF2930, MF1849; MF2973, MF3004, MF3958, MF2971, MF3025, MF2916, MF3991, MF3031, MF2889, MF2913, MF1847, MF3001, MF3003 and MF1898, or wherein said antibody comprises a heavy chain variable region sequence that differs in at most 15 amino acids from the heavy chain variable region sequences of MF2926, MF2930, MF1849; MF2973, MF3004, MF3958, MF2971, MF3025, MF2916, MF3991, MF3031, MF2889, MF2913, MF1847, MF3001, MF3003 or MF1898; and/or

ii) an ErbB-3 specific heavy chain variable region sequence selected from the group consisting of the heavy chain variable region sequences of MF3178; MF3176; MF3163; MF3099; MF3307; MF6055; MF6056; MF6057; MF6058; MF6059; MF6060; MF6061; MF6062; MF6063; MF6064; MF 6065; MF6066; MF6067; MF6068; MF6069; MF6070; MF6071; MF6072; MF6073 and MF6074, or wherein said antibody comprises a heavy chain variable region sequence that differs in at most 15 amino acids from the heavy chain variable region sequences of MF3178; MF3176; MF3163; MF3099; MF3307; MF6055; MF6056; MF6057; MF6058; MF6059; MF6060; MF6061; MF6062; MF6063; MF6064; MF 6065; MF6066; MF6067; MF6068; MF6069; MF6070; MF6071; MF6072; MF6073 or MF6074.

**14.** The bispecific antibody for use according to any of the preceding claims, wherein the antibody comprises at least the CDR1, CDR2 and CDR3 sequences of the ErbB-2 specific heavy chain variable region MF3958 and the antibody comprises at least the CDR1, CDR2 and CDR3 sequences of the ErbB-3 specific heavy chain variable region MF3178.

**15.** The bispecific antibody for use according to any one of the preceding claims, wherein said first antigen binding site and said second antigen binding site comprise a light chain variable region comprising the IgVKl-39 gene segment, most preferably the rearranged germline human kappa light chain IgVKl-39*01/IGJKl*01.

**16.** The bispecific antibody for use according to any one of the preceding claims, wherein said first antigen binding site and said second antigen binding site comprise a light chain variable region comprising a CDR1 having the sequence (RASQSISSYLN), a CDR2 having the sequence (AASSLQS), and a CDR3 having the sequence (QQSYSTPPT).

**17.** A method for the treatment of a subject having an ErbB-2/ErbB-3 positive tumor, the method comprising administering to the individual in need thereof a bispecific antibody comprising a first antigen-binding site that binds ErbB-2 and a second antigen-binding site that binds ErbB-3, wherein the antibody can reduce a ligand-induced receptor function of ErbB-3 on a ErbB-2 and ErbB-3 positive cell, for use in the treatment of, wherein said ErbB-2/ErbB-3 positive tumor has at least 150,000 ErbB-2 cell-surface receptors per cell and wherein said subject has not previously been treated with a ErbB-2 specific therapy or with a ErbB-3 specific therapy.

Figure 1

```
                                                   CDR1                         CDR2
       1        10        20        30                                   50        60
MF3178 QVQLVQSGAEVKKPGASVKVSCKASGYTFT GYYMH WVRQAPGQGLEWMG WINPNSGGTNYAQKFQG
MF6055 .........D.................... ..... ........A..... ....S.......K....
MF6056 .........D..........T......... ..... ........A..... ....S.......K....
MF6057 .........D..........T......... ..... .............. ....Q............
MF6058 .........D..........T......... ..... ........A..... ....Q.......K....
MF6059 .............................. ..... .............. ....G..S.........
MF6060 .........D.................... ..... ........A..... ....Q.......K....
MF6061 .............................. ..... .............. ....Q..........K.
MF6062 .............................. ..... .............. ....G..S.........
MF6063 .............................. ..... .............. ....Q.......K....
MF6064 .............................. ..... .......K...... ....Q............
MF6065 .............................. S.... .............. ....QG.S.........
MF6066 .............................. ..... .............. ....Q..S.........
MF6067 .............................. ..... .............. ....Q............
MF6068 .............................. ..... .............. ....Q............
MF6069 .............................. ..... .............. ....Q............
MF6070 .............................. S.... .............. ....SG.S.........
MF6071 .............................. ..... .............. ....S..S.........
MF6072 .............................. ..... .............. ....S............
MF6073 .............................. ..... .............. ....S............
MF6074 .............................. ..... .............. ....S............

                                                    CDR3
          70        80        90          100       110       120
MF3178 RVTMTRDTSISTAYMELSRLRSDDTAVYYCAR DHGSRHFWSYWGFDY WGQGTLVTVSS
MF6055 ......E..T................T..... ............... ...........
MF6056 ..S...E..T.....Q..........T..... ............... ...........
MF6057 ...............Q................ ............... ...........
MF6058 ..S...E..T.....Q..........T..... ............... ...........
MF6059 ................................ ............... ...........
MF6060 ......E..T................T..... ............... ...........
MF6061 .........T...................... ............... ...........
MF6062 .........T...................... ............... ...........
MF6063 .........T...................... ............... ...........
MF6064 .........T...................... ............... ...........
MF6065 .........T..V.........E......... ............... ...........
MF6066 .........T........S...E......... ............... ...........
MF6067 .........T..V.....S............. ............... ...........
MF6068 .........T...................... ............... ...........
MF6069 ................................ ............... ...........
MF6070 .........T..V.........E......... ............... ...........
MF6071 .........T........S...E......... ............... ...........
MF6072 .........T..V.....S............. ............... ...........
MF6073 .........T...................... ............... ...........
MF6074 ................................ ............... ...........
```

Fig. 2

A

B

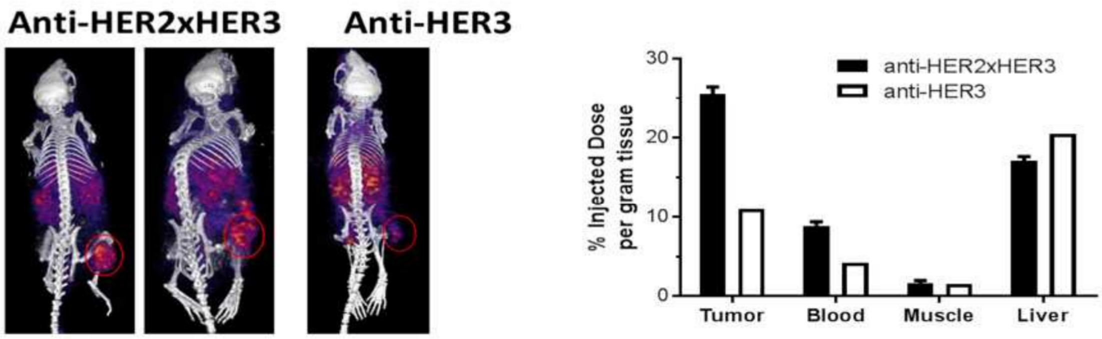
Anti-HER2xHER3
Anti-HER3
% Injected Dose per gram tissue
30
20
10
0
anti-HER2xHER3
anti-HER3
Tumor
Blood
Muscle
Liver

Fig 2. continued

C.

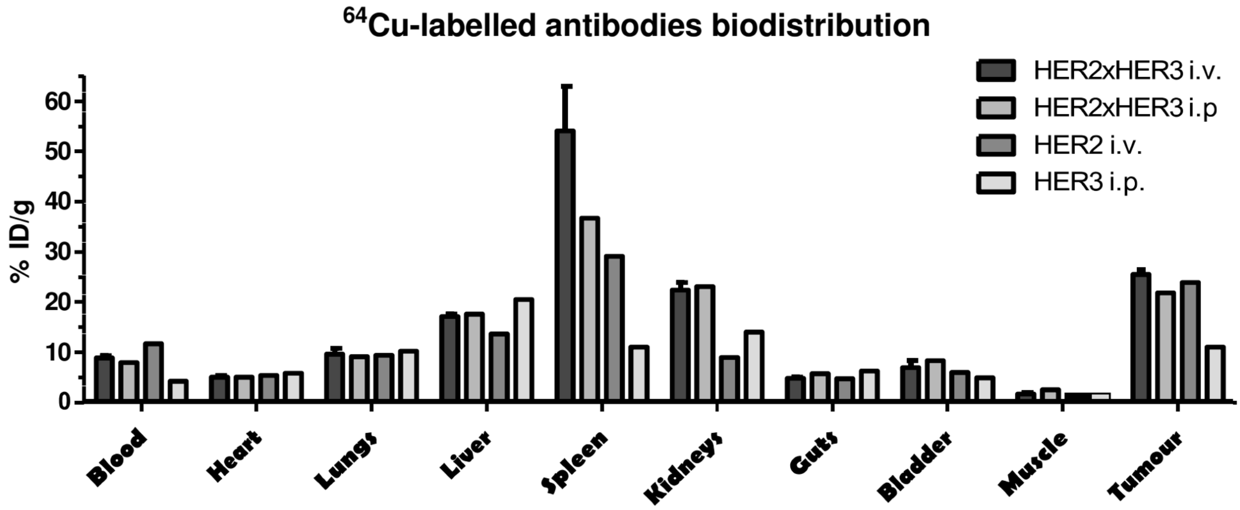

Figure 3

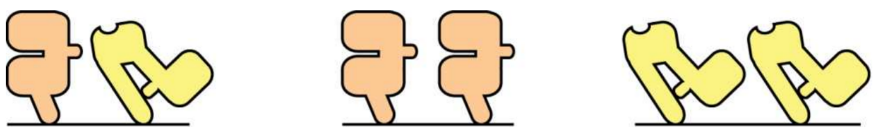

Figure 4

A-B.

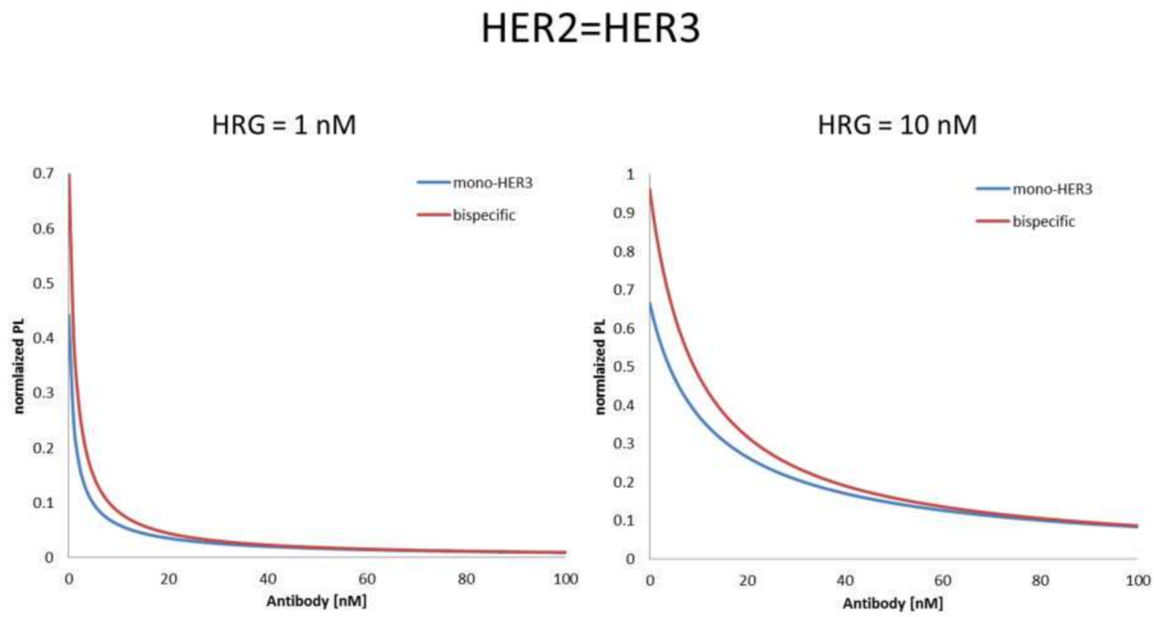
HER2=HER3
HRG = 1 nM
HRG = 10 nM
mono-HER3
bispecific
normlaized PL
Antibody [nM]
0.7
0.6
0.5
0.4
0.3
0.2
0.1
0
1
0.9
0.8
20
40
60
80
100

Figure 4

C-D

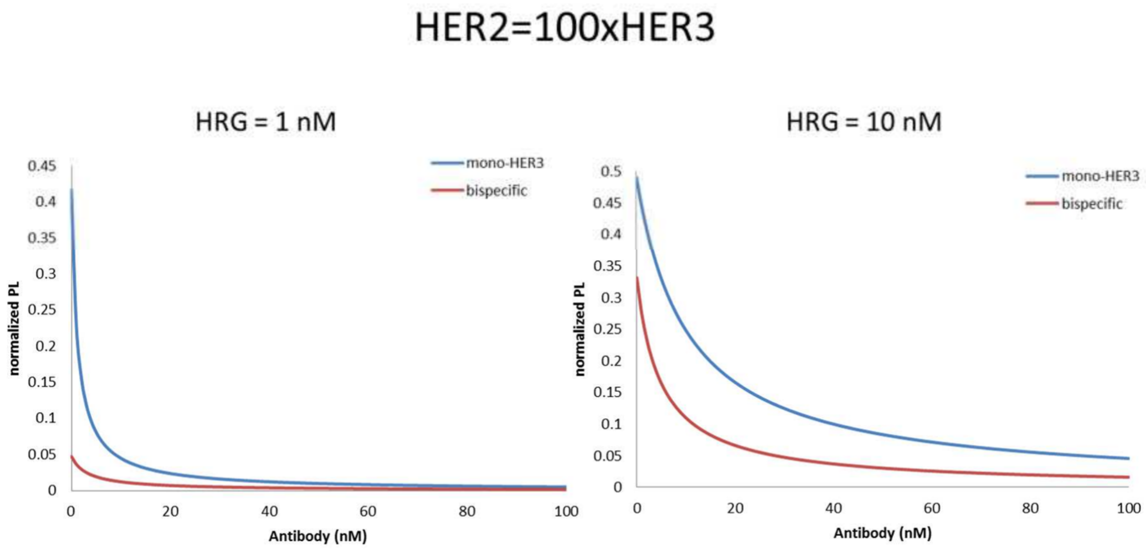
HER2=100xHER3
HRG = 1 nM
mono-HER3
bispecific
normalized PL
0.45
0.4
0.35
0.3
0.25
0.2
0.15
0.1
0.05
0
0
20
40
60
80
100
Antibody (nM)
HRG = 10 nM
mono-HER3
bispecific
normalized PL
0.5
0.45
0.4
0.35
0.3
0.25
0.2
0.15
0.1
0.05
0
0
20
40
60
80
100
Antibody (nM)

Figure 5 A

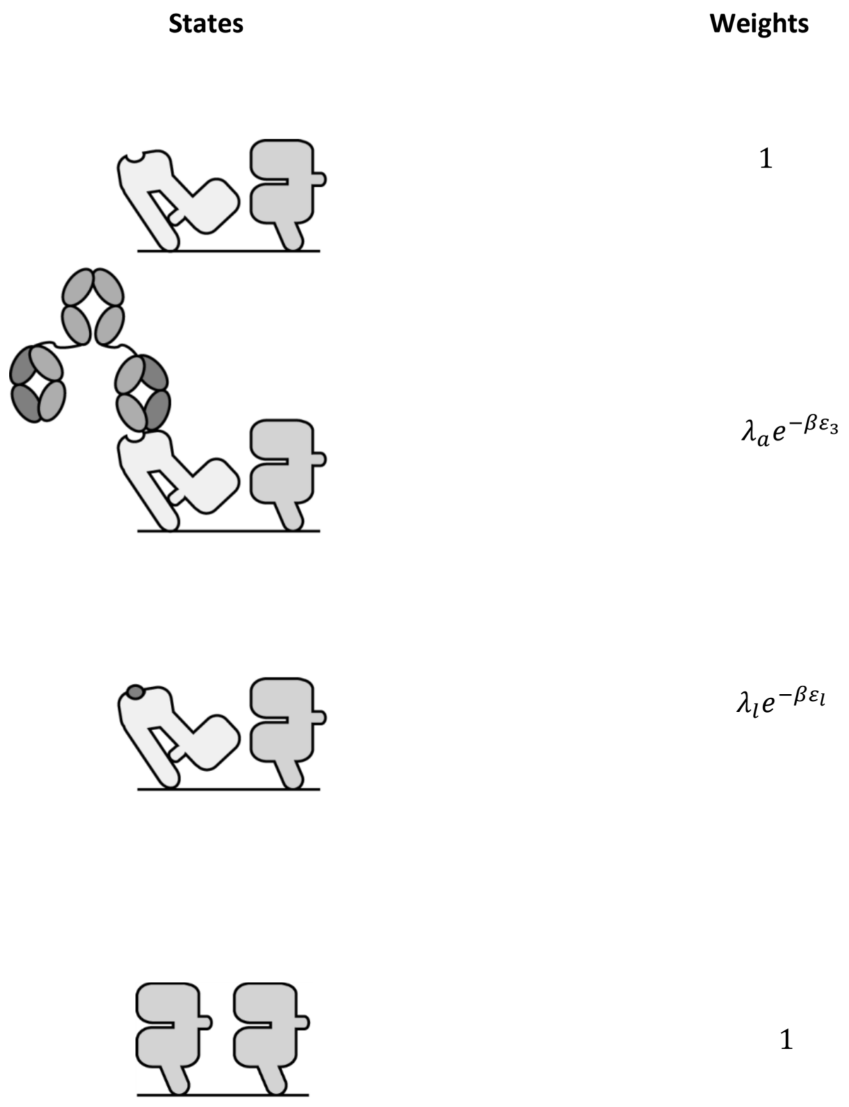
States
Weights
1
$\lambda_a e^{-\beta\varepsilon_3}$
$\lambda_l e^{-\beta\varepsilon_l}$
1

| States | Weights |
|---|---|
| 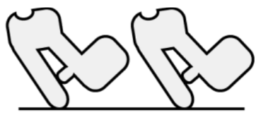 | $1$ |
| 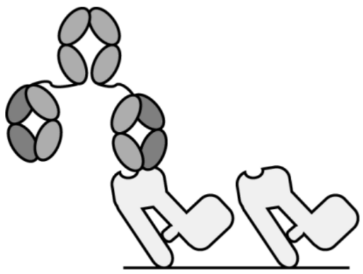 | $2\lambda_a e^{-\beta\varepsilon_3}$ |
| 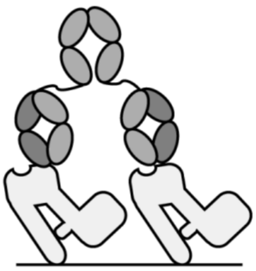 | $\lambda_a e^{-2\beta\varepsilon_3}$ |
| 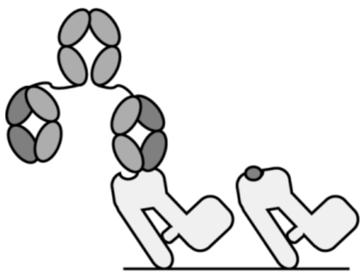 | $2\lambda_a\lambda_l e^{-\beta(\varepsilon_3+\varepsilon_l)}$ |
| 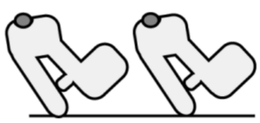 | $\lambda_l^2 e^{-2\beta\varepsilon_l}$ |
| 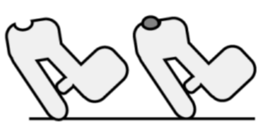 | $2\lambda_l e^{-\beta\varepsilon_l}$ |
| 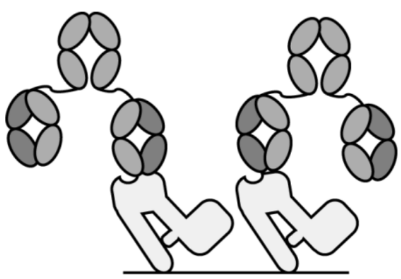 | $\lambda_a^2 e^{-2\beta\varepsilon_3}$ |

**Figure 5B**

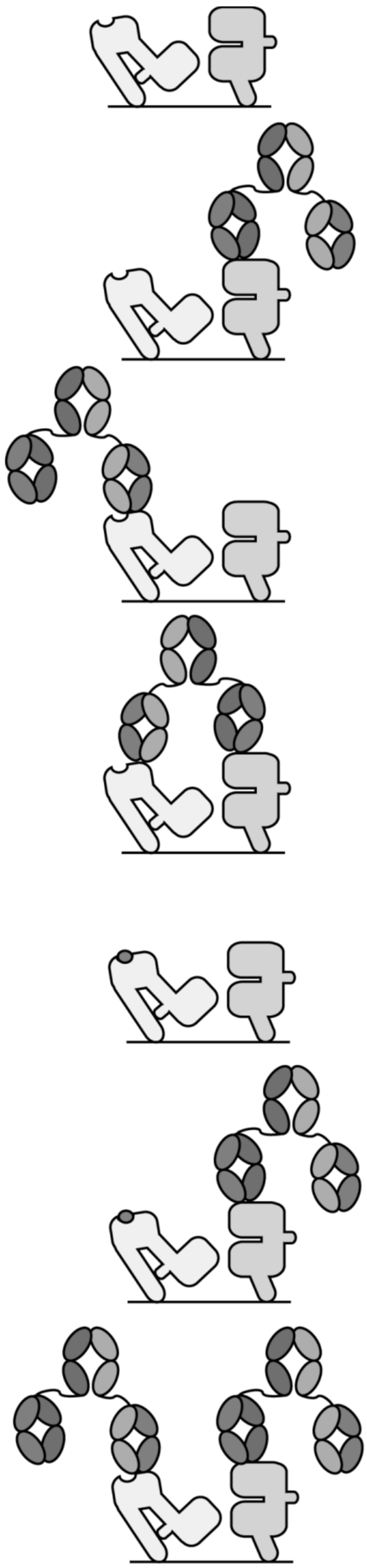

$1$

$\lambda_a e^{-\beta\varepsilon_2}$

$\lambda_a e^{-\beta\varepsilon_3}$

$\lambda_a e^{-\beta(\varepsilon_3+\varepsilon_2)}$

$\lambda_l e^{-\beta\varepsilon_l}$

$\lambda_l \lambda_a e^{-\beta(\varepsilon_l+\varepsilon_2)}$

$\lambda_a^2 e^{-\beta(\varepsilon_3+\varepsilon_2)}$

| **States** | **Weights** |
| --- | --- |
| 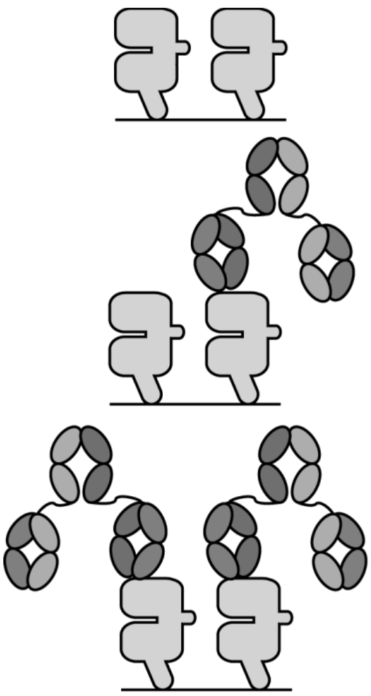 | $1$<br>$\lambda_a e^{-\beta\varepsilon_2}$<br>$\lambda_a^2 e^{-2\beta\varepsilon_2}$ |

| **States** | **Weights** |
|---|---|
| 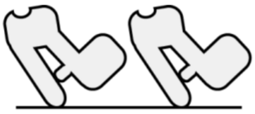 | 1 |
| 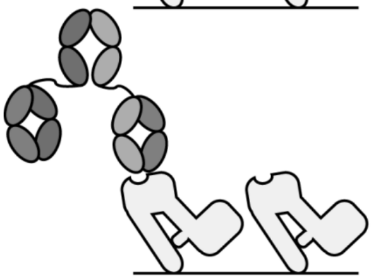 | $2\lambda_a e^{-\beta\varepsilon_3}$ |
| 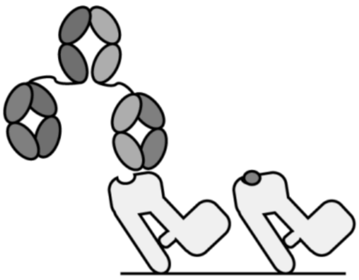 | $2\lambda_a\lambda_l e^{-\beta(\varepsilon_3+\varepsilon_l)}$ |
| 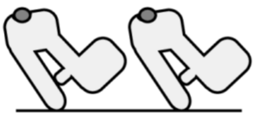 | $\lambda_l^2 e^{-2\beta\varepsilon_l}$ |
| 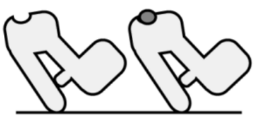 | $2\lambda_l e^{-\beta\varepsilon_l}$ |
| 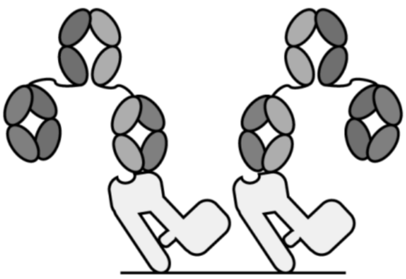 | $\lambda_a^2 e^{-2\beta\epsilon_3}$ |

Figure 6

A

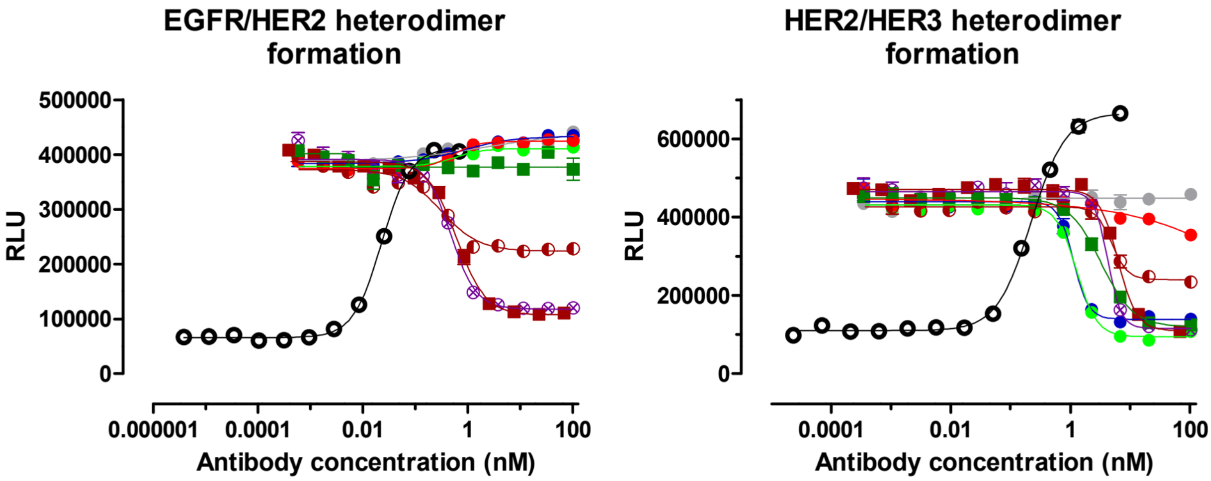
EGFR/HER2 heterodimer formation
RLU
500000
400000
300000
200000
100000
0
0.000001
0.0001
0.01
1
100
Antibody concentration (nM)
HER2/HER3 heterodimer formation
RLU
600000
400000
200000
0
0.0001
0.01
1
100
Antibody concentration (nM)

Figure 6

B

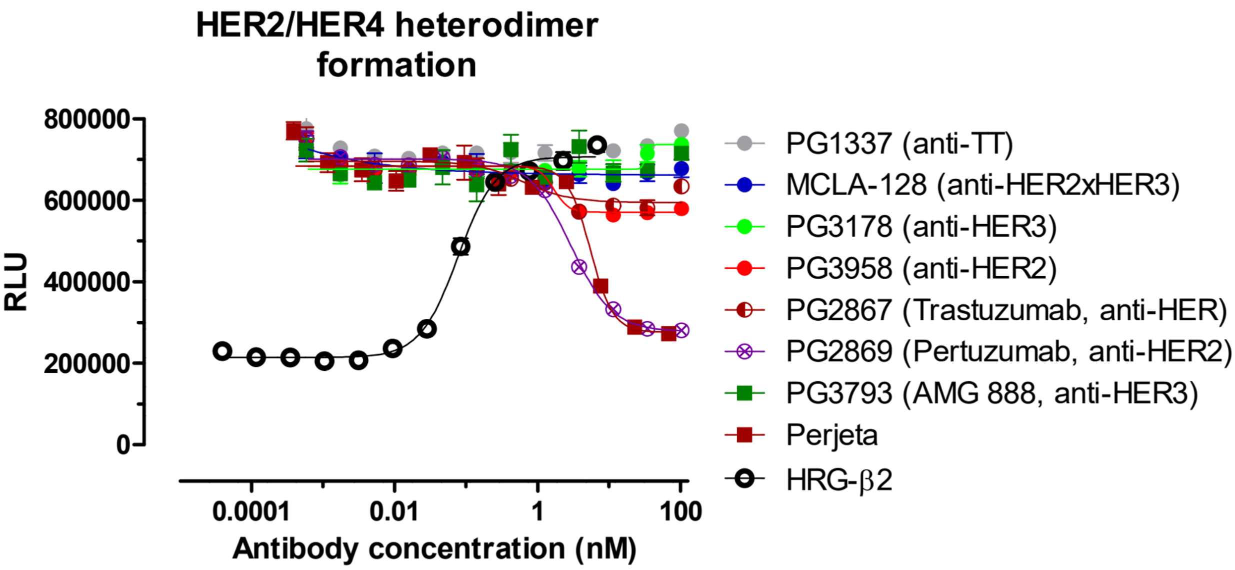
HER2/HER4 heterodimer formation
800000
600000
400000
200000
0
RLU
0.0001
0.01
1
100
Antibody concentration (nM)
PG1337 (anti-TT)
MCLA-128 (anti-HER2xHER3)
PG3178 (anti-HER3)
PG3958 (anti-HER2)
PG2867 (Trastuzumab, anti-HER)
PG2869 (Pertuzumab, anti-HER2)
PG3793 (AMG 888, anti-HER3)
Perjeta
HRG-β2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 17164382
- WO 2015130173 A
- US 20030078385 A, Arathoon
- NL 2013050294 W
- WO 2013157954 A1


### Non-patent literature cited in the description


- **CHERNOMORIDIK et al.** *Mol Imaging.*, August 2010, vol. 9 (4), 192-200
- **ARDESHIRPOUR et al.** *Technol Cancer Res Treat.*, October 2014, vol. 13 (5), 427-434
- **VAN DER WONING** ; **VAN ZOELEN**. *Biochem Biophys Res Commun.*, 09 January 2009, vol. 378 (2), 285-9
- **MCDONAGH et al.** *Mol Cancer Ther*, 2012, vol. 11, 582
- **SHAMES et al.** *PLOS ONE*, February 2013, vol. 8 (2), 1-10
- **YONESAKA et al.** *Sci.transl.Med.*, 2011, vol. 3 (99), 1-11
- **JUNTTILA, T. T.** ; **K. PARSONS et al.** Superior In vivo Efficacy of Afucosylated Trastuzumab in the Treatment of HER2-Amplified Breast Cancer.. *Cancer Research*, 2010, vol. 70 (11), 4481-4489
- **LANDGRAF, R**. *Breast Cancer Res.*, 2007, vol. 9 (1), 202
- **PATERSON**. *Dalton Transactions*, 2014
- **YARDEN Y** ; **PINES G.** The ERBB network: at last, cancer therapy meets systems biology. *Nat Rev Cancer*, 12 July 2012, vol. 12 (8), 553-63
- **WILSON TR** ; **FRIDLYAND J** ; **YAN Y** ; **PENUEL E** ; **BURTON L** ; **CHAN E** ; **PENG J** ; **LIN E** ; **WANG Y** ; **SOSMAN J**. Widespread potential for growth-factor-driven resistance to anticancer kinase inhibitors. *Nature*, 26 July 2012, vol. 487 (7408), 505-9
- **BALKO JM** ; **MILLER TW** ; **MORRISON MM** ; **HUTCHINSON K** ; **YOUNG C** ; **RINEHART C** ; **SÁNCHEZ V** ; **JEE D** ; **POLYAK K** ; **PRAT A**. The receptor tyrosine kinase ErbB3 maintains the balance between luminal and basal breast epithelium. *Proc Natl Acad Sci U S A.*, 03 January 2012, vol. 109 (1), 221-6
- **ZHANG H** ; **BEREZOV A** ; **WANG Q** ; **ZHANG G** ; **DREBIN J** ; **MURALI R** ; **GREENE MI**. ErbB receptors: from oncogenes to targeted cancer therapies. *J Clin Invest.*, August 2007, vol. 117 (8), 2051-8
- **SERGINA NV** ; **RAUSCH M** ; **WANG D** ; **BLAIR J** ; **HANN B** ; **SHOKAT KM** ; **MOASSER MM**. Escape from HER-family tyrosine kinase inhibitor therapy by the kinase-inactive HER3. *Nature*, 25 January 2007, vol. 445 (7126), 437-41
- **JUNTTILA TT** ; **AKITA RW** ; **PARSONS K** ; **FIELDS C** ; **LEWIS PHILLIPS GD** ; **FRIEDMAN LS** ; **SAMPATH D** ; **SLIWKOWSKI MX**. Ligand-independent HER2/HER3/PI3K complex is disrupted by trastuzumab and is effectively inhibited by the PI3K inhibitor GDC-0941. *Cancer Cell*, 05 May 2009, vol. 15 (5), 429-40
- **OCANA A** ; **VERA-BADILLO F** ; **SERUGA B** ; **TEMPLETON A** ; **PANDIELLA A** ; **AMIR E.** HER3 overexpression and survival in solid tumors: a meta-analysis. *J Natl Cancer Inst.*, 20 February 2013, vol. 105 (4), 266-73
- **MERCHANT et al.** *Nature Biotechnology*, July 1998, vol. 16, 677-681